# EUROPÄISCHE PATENTANMELDUNG

(11) **EP 2 241 306 A2**
(43) Veröffentlichungstag der Anmeldung: **20.10.2010**
(21) Anmeldenummer: 09178676.4
(22) Anmeldetag: 10.12.2009
(51) Int. Cl.: A61K 8/64, A61Q 19/08

(54) **Zusamensetzung mit antioxidativ wirksamen Peptiden**

(30) Priorität: 11.12.2008 DE 102008061044
(71) Anmelder: Henkel AG & Co. KGaA, 40589 Düsseldorf (DE)
(72) Erfinder: Janßen, Frank, 41470, Neuss (DE)

(57) **Zusammenfassung**

Die Erfindung betrifft Zusammensetzung, enthaltend mindestens ein Peptid, ausgewählt aus Peptiden, welche eine Länge von drei bis sechzehn Aminosäuren besitzen und mindestens eine positive Nettoladung (bei pH 7,2) aufweisen und mindestens zwei Aminosäuren mit aromatischen Seitenketten aufweisen, von denen mindestens eine Aminosäure ausgewählt ist aus Tyr, 2'-Methyltyrosin, 6'-Methyltyrosin, 2',6'-Dimethyltyrosin (Dmt), N,2',6'-Trimethyltyrosin (Tmt) oder 2'-Hydroxy-6'-Methyltyrosin (Hmt); sowie 0,001 bis 95 Gew.-% Wasser sowie die Verwendung mindestens eines solchen Peptids zur Bekämpfung der Hautalterung, zum Schutz der Haut vor Schäden durch ultraviolette und/oder infrarote Strahlung, zur Verbesserung der Elastizität der Haut und/oder zur Verringerung und/oder Verhinderung der Faltenbildung.

SEQUENCE LISTING

<110>  Henkel AG & Co. KGaA

<120>  Zusammensetzung mit antioxidativ wirksamen Peptiden

<130>  H 08156 EP

<150>  DE 102008061044.5
<151>  2008-12-11

<160>  22

<170>  PatentIn version 3.3

<210>  1
<211>  4
<212>  PRT
<213>  Artificial

<220>
<223>  Artificial

<400>  1

Gly Gln Pro Arg
1


<210>  2
<211>  4
<212>  PRT
<213>  Artificial

<220>
<223>  N-Palmitoyl

<400>  2

Gly Gln Pro Arg
1


<210>  3
<211>  4
<212>  PRT
<213>  Artificial

<220>
<223>  Artificial

<400>  3

Ala Gln Thr Arg
1

<210>  4
<211>  4
<212>  PRT
<213>  Artificial

<220>
<223>  tetrapeptide


<220>
<221>  misc_feature
<222>  (1)..(1)
<223>  Xaa chosen from Gly and amino acids that can replace Gly,
       preferably bAla, Ala, Val , Leu, Pro, Sar (Sarcosin), Ile.

<220>
<221>  misc_feature
<222>  (2)..(2)
<223>  Xaa chosen from Gln and amino acids that can replace Gln,
       preferably Asn, Lys, Orn, 5-Hydroxyprolin, Citrullin, Canavanin.

<220>
<221>  misc_feature
<222>  (4)..(4)
<223>  Xaa chosen from Pro or Arg and amino acids that can replace Pro
       or Arg, Arg replacing amino acids are preferably Pro, Lys His,
       Desmosin or Isodesmosin.

<400>  4

Xaa Xaa Arg Xaa
1


<210>  5
<211>  4
<212>  PRT
<213>  Artificial

<220>
<223>  Artificial Sequence


<220>
<221>  misc_feature
<222>  (1)..(1)
<223>  Xaa chosen from Gly and amino acids that can replace Gly,
       preferably bAla, Ala, Val , Leu, Pro, Sar (Sarcosin), Ile.

<220>
<221>  misc_feature
<222>  (2)..(2)
<223>  Xaa chosen from Gln and amino acids that can replace Gln,
       preferably Asn, Lys, Orn, 5-Hydroxyprolin, Citrullin, Canavanin.

<220>
<221>  misc_feature

<222>  (3)..(3)
<223>  Xaa chosen from Pro or Arg and amino acids that can replace Pro
       or Arg, Arg replacing amino acids are preferably Pro, Lys His,
       Desmosin or Isodesmosin.

<400>  5

Xaa Xaa Xaa Pro
1


<210>  6
<211>  4
<212>  PRT
<213>  Artificial

<220>
<223>  Artificial Sequence


<220>
<221>  misc_feature
<222>  (1)..(1)
<223>  Xaa chosen from Gly and amino acids that can replace Gly,
       preferably bAla, Ala, Val , Leu, Pro, Sar (Sarcosin), Ile.

<220>
<221>  misc_feature
<222>  (2)..(2)
<223>  Xaa chosen from Gln and amino acids that can replace Gln,
       preferably Asn, Lys, Orn, 5-Hydroxyprolin, Citrullin, Canavanin.

<220>
<221>  misc_feature
<222>  (4)..(4)
<223>  Xaa chosen from Pro or Arg and amino acids that can replace Pro
       or Arg, Arg replacing amino acids are preferably Pro, Lys His,
       Desmosin or Isodesmosin.

<400>  6

Xaa Xaa Pro Xaa
1


<210>  7
<211>  4
<212>  PRT
<213>  Artificial

<220>
<223>  Artificial Sequence


<220>
<221>  misc_feature
<222>  (1)..(1)

<223>  Xaa chosen from Gly and amino acids that can replace Gly,
       preferably bAla, Ala, Val , Leu, Pro, Sar (Sarcosin), Ile.

<220>
<221>  misc_feature
<222>  (2)..(2)
<223>  Xaa chosen from Gln and amino acids that can replace Gln,
       preferably Asn, Lys, Orn, 5-Hydroxyprolin, Citrullin, Canavanin.

<400>  7

Xaa Xaa Pro Arg
1


<210>  8
<211>  4
<212>  PRT
<213>  Artificial

<220>
<223>  Artificial Sequence


<220>
<221>  misc_feature
<222>  (1)..(1)
<223>  Xaa chosen from Gly and amino acids that can replace Gly,
       preferably bAla, Ala, Val , Leu, Pro, Sar (Sarcosin), Ile.

<220>
<221>  misc_feature
<222>  (2)..(2)
<223>  Xaa chosen from Gln and amino acids that can replace Gln,
       preferably Asn, Lys, Orn, 5-Hydroxyprolin, Citrullin, Canavanin.

<220>
<221>  misc_feature
<222>  (3)..(3)
<223>  Xaa chosen from Pro or Arg and amino acids that can replace Pro
       or Arg, Arg replacing amino acids are preferably Pro, Lys His,
       Desmosin or Isodesmosin.

<400>  8

Xaa Xaa Xaa Arg
1


<210>  9
<211>  4
<212>  PRT
<213>  Artificial

<220>
<223>  Artificial Sequence

<400>  9

Val Val Arg Pro
1

<210>  10
<211>  5
<212>  PRT
<213>  Artificial

<220>
<223>  preferably with N-Acyl or ester-bond

<400>  10

Lys Thr Thr Lys Ser
1               5

<210>  11
<211>  5
<212>  PRT
<213>  Artificial

<220>
<223>  Artificial Sequence

<220>
<221>  MOD_RES
<222>  (1)..(1)
<223>  N-Palmitoyl-Lys

<400>  11

Lys Thr Thr Lys Ser
1               5

<210>  12
<211>  5
<212>  PRT
<213>  Artificial

<220>
<223>  Artificial Sequence

<220>
<221>  MOD_RES
<222>  (1)..(1)
<223>  N-Palmitoyl-Tyr

<400>  12

Tyr Gly Gly Phe Met

1               5


<210>  13
<211>  5
<212>  PRT
<213>  Artificial

<220>
<223>  Artificial Sequence

<400>  13

Val Val Arg Pro Pro
1               5


<210>  14
<211>  5
<212>  PRT
<213>  Artificial

<220>
<223>  Artificial Sequence


<220>
<221>  MOD_RES
<222>  (1)..(1)
<223>  N-Palmitoyl-Tyr

<400>  14

Tyr Gly Gly Phe Leu
1               5


<210>  15
<211>  5
<212>  PRT
<213>  Artificial

<220>
<223>  Artificial Sequence


<220>
<221>  MOD_RES
<222>  (1)..(1)
<223>  N-Benzyloxycarbonyl-Gly

<400>  15

Gly Pro Phe Pro Leu
1               5

<210>  16
<211>  5
<212>  PRT
<213>  Artificial

<220>
<223>  Artificial Sequence

<400>  16

Gly Pro Phe Pro Leu
1               5


<210>  17
<211>  6
<212>  PRT
<213>  Artificial

<220>
<223>  Artificial Sequence

<400>  17

Val Gly Val Ala Pro Gly
1               5


<210>  18
<211>  6
<212>  PRT
<213>  Artificial

<220>
<223>  Artificial Sequence


<220>
<221>  MOD_RES
<222>  (1)..(1)
<223>  N-Palmitoyl-VAL

<400>  18

Val Gly Val Ala Pro Gly
1               5


<210>  19
<211>  6
<212>  PRT
<213>  Artificial

<220>
<223>  Peptide 02 Vincience

<400>  19

Ala Asp Leu Lys Pro Thr
1               5


<210>  20
<211>  6
<212>  PRT
<213>  Artificial

<220>
<223>  MOD_RES


<220>
<221>  MOD_RES
<222>  (4)..(4)
<223>  Xaa=Methylnorleucin

<220>
<221>  MOD_RES
<222>  (5)..(5)
<223>  Xaa=Homophenylalanin

<400>  20

Ala Arg His Xaa Xaa Trp
1               5


<210>  21
<211>  6
<212>  PRT
<213>  Artificial

<220>
<223>  Hexapeptide-1

<400>  21

Ala Arg His Leu Phe Trp
1               5


<210>  22
<211>  6
<212>  PRT
<213>  Artificial

<220>
<223>  Artificial Sequence

<400>  22

Val Val Arg Pro Pro Pro
1               5

## Beschreibung

Die Erfindung betrifft Zusammensetzung, enthaltend mindestens ein Peptid, ausgewählt aus Peptiden, welche eine Länge von drei bis sechzehn Aminosäuren besitzen und mindestens eine positive Nettoladung (bei pH 7,2) aufweisen und mindestens zwei Aminosäuren mit aromatischen Seitenketten aufweisen, von denen mindestens eine Aminosäure ausgewählt ist aus Tyr, 2'-Methyltyrosin, 6'-Methyltyrosin, 2',6'-Dimethyltyrosin (Dmt), N,2',6'-Trimethyltyrosin (Tmt) oder 2'-Hydroxy-6'-Methyltyrosin (Hmt); sowie 0,001 bis 95 Gew.-% Wasser sowie die Verwendung mindestens eines solchen Peptids zur Bekämpfung der Hautalterung, zum Schutz der Haut vor Schäden durch ultraviolette und/oder infrarote Strahlung, zur Verbesserung der Elastizität der Haut und/oder zur Verringerung und/oder Verhinderung der Faltenbildung,

Mittel und Behandlungsmethoden zur Behandlung reifer oder intrinsisch oder extrinsisch gealterter oder lichtgeschädigter Haut sind eine bedeutende kosmetische Herausforderung. Bei diesen Mitteln besteht ein Bedarf nach Inhaltsstoffen, die den Zubereitungen weiteren Nutzen verleihen, sei es aus anwendungstechnischer Sicht, aus herstelltechnischer Sicht oder aus Sicht des Verbrauchers, der beispielsweise aufgrund bestimmter Inhaltsstoffe ein verbessertes subjektives Empfinden dem kosmetischen Mittel gegenüber entwickelt. Der Schwerpunkt der Leistung eines Inhaltsstoffes kann dabei je nach kosmetischem Mittel auch unterschiedlich sein. So kann ein und derselbe Stoff in einem Shampoo oder einer Spülung pflegende Eigenschaften für die behandelten Haare bewirken, während er in einem Haarfärbemittel darüber hinaus die Anfärbung der Kopfhaut verringert oder die zum Teil aggressive Wirkung des Färbemittels kompensiert.

Die Altershaut wird kosmetisch in erster Linie mit Vitamin A-Derivaten oder Hydroxysäuren behandelt, die über eine Stimulation der Proliferation der Basalzellen in der Epidermis zu einer Verdickung der Epidermis und damit Glättung der Haut führen.

Retinoide greifen in den Metabolismus der Hautzellen ein und bewirken neben der Anregung der Proliferation und Differenzierung der epidermalen Keratinozyten die Steigerung der Collagenproduktion durch Fibroblasten. Darüber hinaus soll Retinol die Bildung von Collagen verdauenden Enzymen reduzieren (New Scientist 2031, 42-46, 1996). Retinsäure besitzt jedoch teratogene Eigenschaften und darf nur in verschreibungspflichtigen Pharmaka eingesetzt werden. Der Einsatz von Retinol in kosmetischen und pharmazeutischen topischen Praeparaten ist aus mehreren Gründen als problematisch zu betrachten. So weist Retinol eine relative hohe Phototoxizität auf und kann deshalb nur in geringen Konzentrationen in Zusammensetzungen zur Anwendung am Menschen eingesetzt werden. Darüber hinaus wird Retinol unter Einwirkung von Wärme und/oder Licht leicht oxidativ abgebaut und ist in kosmetischen und pharmazeutischen Formulierungen schwierig zu stabilisieren.

Hydroxysäuren sind in den für die Behandlung der Hautalterung notwendigen Konzentrationen häufig schlecht verträglich, insbesondere für die Behandlung empfindlicher Haut und für die Behandlung der Haut im Augenbereich.

Neuere Ansätze bestehen darin, die Proteine, die in der trockenen oder der Altershaut fehlen oder vermindert vorkommen, gezielt zu substituieren bzw. indirekt in die Stoffwechselprozesse einzugreifen, die bei trockener Haut oder mit zunehmendem Alter gestört sind, um diese zu normalisieren. Als Beispiel sei hier die Stimulation der Collagensynthese mit dem Zweck der Faltenreduzierung angeführt. Weiterhin werden beispielsweise Laminin, Substanzen zur Verlängerung der Lebenszeit von Hautzellen und bestimmte Extrakte zur Stimulierung der epidermalen Differenzierung eingesetzt. Hierbei handelt es sich teilweise jedoch um pharmakologisch wirksame Substanzen mit hohem Nebenwirkungspotenzial.

Um eine besonders starke Hautalterung zu bekämpfen und zu verhindern, müssen die Zellen der Haut vor den Einflüssen von UV-Strahlung geschützt werden. Weiterhin müssen die Zellen in die Lage versetzt werden, solche Schäden, die durch UV-Strahlung hervorgerufen werden und somit zu einer stärkeren extrinsischen Hautalterung führen, selbst zu reparieren bzw. zu vermindern und so die Hautalterung einzudämmen.

In den internationalen Patentanmeldungen W02005072295 und W02004070054 sind Peptide beschrieben, die zur Behandlung von neurodegenerativen Krankheiten u.a. wie Amyotrophe Laterale Sklerose, Friedrichs Ataxie, Huntington, Parkinson oder Alzheimer eingesetzt werden sollen. Eine topische Applikation auf der Haut oder eine Verwendung der beschriebenen Peptide zur Bekämpfung der Hautalterung, zum Schutz der Haut vor Schäden durch ultraviolette und/oder infrarote Strahlung, zur Verbesserung der Elastizität der Haut und/oder zur Verringerung und/oder Verhinderung der Faltenbildung, wurde nicht beschrieben.

Eine Aufgabe der vorliegenden Erfindung ist es, gut verträgliche Wirkstoffe und Zusammensetzungen, die diese gut verträglichen Wirkstoffe enthalten, zur Verfügung zu stellen, um die Hautalterung zu bekämpfen.

Eine weitere Aufgabe der vorliegenden Erfindung ist es, gut verträgliche Wirkstoffe und Zusammensetzungen, die diese gut verträglichen Wirkstoffe enthalten, zur Verfügung zu stellen, um sie zum Schutz der Haut vor Schäden durch ultraviolette und/oder infrarote Strahlung einzusetzen.

Eine weitere Aufgabe der vorliegenden Erfindung ist es, gut verträgliche Wirkstoffe und Zusammensetzungen, die diese gut verträglichen Wirkstoffe enthalten, zur Verfügung zu stellen, um die Elastizität der Haut zu verbessern.

Eine weitere Aufgabe der vorliegenden Erfindung ist es, gut verträgliche Wirkstoffe und Zusammensetzungen, die diese gut verträglichen Wirkstoffe enthalten, zur Verfügung zu stellen, um die Faltenbildung zu verhindern und/oder zu verringern.

Überraschend wurde gefunden, dass Zusammensetzungen enthaltend enthaltend mindestens ein Peptid, ausgewählt aus Peptiden, welche
a) eine Länge von drei bis sechzehn Aminosäuren besitzen,
b) mindestens eine positive Nettoladung (bei pH 7,2) aufweisen und
c) mindestens zwei Aminosäuren mit aromatischen Seitenketten aufweisen, von denen mindestens eine Aminosäure ausgewählt ist aus Tyr, 2'-Methyltyrosin, 6'-Methyltyrosin, 2',6'-Dimethyltyrosin (Dmt), N,2',6'-Trimethyltyrosin (Tmt) oder 2'-Hydroxy-6'-Methyltyrosin (Hmt)
und 0,001 bis 95 Gew.-% Wasser die gestellten Aufgaben in hervorragender Weise löst.

Gegenstand der vorliegenden Erfindung ist eine Zusammensetzung enthaltend mindestens ein Peptid, ausgewählt aus Peptiden, welche
a) eine Länge von drei bis sechzehn Aminosäuren besitzen,
b) mindestens eine positive Nettoladung (bei pH 7,2) aufweisen und
c) mindestens zwei Aminosäuren mit aromatischen Seitenketten aufweisen, von denen mindestens eine Aminosäure ausgewählt ist aus Tyr, 2'-Methyltyrosin, 6'-Methyltyrosin, 2',6'-Dimethyltyrosin (Dmt), N,2',6'-Trimethyltyrosin (Tmt) oder 2'-Hydroxy-6'-Methyltyrosin (Hmt)
und 0,001 bis 95 Gew.-% Wasser.

Die erfindungsgemäß einzusetzenden Peptide weisen eine Länge von drei bis sechzehn Aminosäuren auf. Längere Aminosäuren sind nicht in der Lage, die Mitochondrienmembran zu durchqueren und in den Mitochondrien ihre antioxidative Wirkung zu entfalten.

Solche antioxidative Substanzen sind überraschender Weise in der Lage, durch die Zellwände der Mitochondrien in die Mitochondrien selbst zu gelangen und dort den Alterungsprozess positiv zu beeinflussen, insbesondere zu verlangsamen. Überraschenderweise wurde nun gefunden, dass der Einsatz nicht nur systemisch, sondern auch topisch erfolgen kann.

Eine verbesserte Verfügbarkeit von Substanzen mit antioxidativer Wirkung in den Mitochondrien der Fibroblasten führt zu einer verlängerten Lebenszeit der Fibroblasten, die die extracelluläre Matrix herstellen. Die Reduktion der extrazellulären Matrix in der Dermis führt zu Faltenbildung, Verlust der Elastizität der Haut sowie zu allgemeiner Hautalterung. Durch eine verbesserte Versorgung der Mitochondrien (als Kraftwerke der Fibroblasten) mit antioxidativen Substanzen kann die volle Funktionsfähigkeit der einzelnen Zelle länger gewährleistet werden und die negativen Begleiterscheinungen des Älterwerdens können herausgezögert werden. Somit sind die erfindungsgemäßen Zusammensetzungen als Anti-Aging-Mittel für die Haut besonders gut geeignet.

Gemäß einer bevorzugten Ausführungsform weist das erfindungsgemäß einzusetzende Peptid eine Länge von vier bis zwölf, bevorzugt von vier bis acht Aminosäuren auf.

Weiterhin müssen die Peptide bei pH 7,2 (als einem physiologischen pH-Wert) mindestens eine positive Nettoladung aufweisen.

Im erfindungsgemäßen Zusammenhang ist hierbei die positive Nettoladung in einer wässrigen Lösung gemeint. Die wässrige Lösung, in der eine positive Nettoladung ggf. bestimmt werden soll, darf maximal eine physiologisch verträgliche lonenstärke aufweisen. Zu hohe Salzkonzentrationen oder nicht wässrige bzw. Mischsysteme aus wässriger Phase mit hydrophoberen Lösungsmitteln, z.B. Alkoholen können die Protonierbarkeit bzw. die pK-Werte verändern.

Die positive Nettoladung bei pH 7,2 kann entweder dadurch erzeugt werden, dass das erfindungsgemäß einzusetzende Peptid nur eine Aminosäure aufweist, die bei einem pH-Wert von 7,2 eine Seitenkette der Aminosäure mit einer positiven Ladung aufweist. An einer solchen Seitenkette befindet sich dabei in der Regel eine funktionelle Gruppe. Diese funktionelle Gruppe muß einen pK-Wert aufweisen, der darin resultiert, dass die funktionelle Gruppe bei pH 7,2 protoniert vorliegt. Geeignete Aminosäure sind beispielsweise die sogenannten basischen essentiellen Aminosäuren Arg, Lys und His.

Eine weitere Möglichkeit, mindestens eine positive Nettoladung zu erzeugen, ist es, dass in dem Peptid sowohl saure Aminosäuren, d.h. mit bei pH 7,2 deprotonierter funktioneller Gruppe in der Seitenketten als auch basische Aminosäuren, d.h. mit bei pH 7,2 protonierter funktioneller Gruppe in der Seitenkette vorhanden sind, wobei mindestens eine basische Aminosäure mehr als saure Aminosäuren vorhanden ist.

Um durch die Zellmembran in die Zelle und dann auch noch in die Mitochondrien zu gelangen, müssen die Peptide sowohl lipophile als auch kationische Eigenschaften aufweisen.

Erfindungsgemäß bevorzugte Peptide sind beispielsweise:
Lys-D-Arg-Tyr-NH₂, Phe-D-Arg-His, D-Tyr-Trp-Lys-NH₂, Trp-D-Lys-Tyr-Arg-NH₂, Tyr-His-D-Gly-Met, Phe-Arg-D-His-Asp, Tyr-D-Arg-Phe-Lys-Glu-NH₂, Met-Tyr-D-Lys-Phe-Arg, D-His-Glu-Lys-Tyr-D-Phe-Arg, Lys-D-Gln-Tyr-Arg-D-Phe-Trp-NH₂,
Phe-D-Arg-Lys-Trp-Tyr-D-Arg-His, Gly-D-Phe-Lys-Tyr-His-D-Arg-Tyr- NH₂, Val-D-Lys-His-Tyr-D-Phe-Ser-Tyr-Arg- NH₂, Trp-Lys-Phe-D-Asp-Arg-Tyr-D-His-Lys, Lys-Trp-D-Tyr-Arg-Asn-Phe-Tyr-D-His-NH₂ Thr-Gly-Tyr-Arg-D-His-Phe-Trp-D-His-Lys, Asp-D-Trp-Lys-Tyr-D-His-Phe-Arg-D-Gly-Lys-NH₂, D-His-Lys-Tyr-D-Phe-Glu-D-Asp-D-His-D-Lys-Arg-Trp-NH₂,
Ala-D-Phe-D-Arg-Tyr-Lys-D-Trp-His-D-Tyr-Gly-Phe, Tyr-D-His-Phe-D-Arg-Asp-Lys-D-Arg-His-Trp-D-His-Phe, Phe-Phe-D-Tyr-Arg-Glu-Asp-D-Lys-Arg-D-Arg-His-Phe-NH₂, Phe-Try-Lys-D-Arg-Trp-His-D-Lys-D-Lys-Glu-Arg-D-Tyr-Thr, Tyr-Asp-D-Lys-Tyr-Phe-D-Lys-D-Arg-Phe-Pro-D-Tyr-His-Lys, Glu-Arg-D-Lys-Tyr-D-Val-Phe-D-His-Trp-Arg-D-Gly-Tyr-Arg-D-Met-NH₂, Arg-D-Leu-D-Tyr-Phe-Lys-Glu-D-Lys-Arg-D-Trp-Lys-D-Phe-Tyr-D-Arg-Gly, D-Glu-Asp-Lys-D-Arg-D-His-Phe-Phe-D-Val-Tyr-Arg-Tyr-D-Tyr-Arg-His-Phe-NH₂, Asp-Arg-D-Phe-Cys-Phe-D-Arg-D-Lys-Tyr-Arg-D-Tyr-Trp-D-His-Tyr-D-Phe-Lys-Phe, His-Tyr-D-Arg-Trp-Lys-Phe-D-Asp-Ala-Arg-Cys-D-Tyr-His-Phe-D-Lys-Tyr-His-Ser-NH₂, Gly-Ala-Lys-Phe-D-Lys-Glu-Arg-Tyr-His-D-Arg-D-Arg-Asp-Tyr-Trp-D-His-Trp-His-D-Lys-Asp und Thr-Tyr-Arg-D-Lys-Trp-Tyr-Glu-Asp-D-Lys-D-Arg-His-Phe-D-Tyr-Gly-Val-Ile-D-His-Arg-Tyr-NH₂.

Gemäß einer besonders bevorzugten Ausführungsform der Erfindung weist das Peptid eine alternierende Abfolge von Aminosäuren mit bei pH 7,2 positiv geladenen Seitenketten und Aminosäuren mit aromatischen Seitenketten auf. Solche Peptide gelangen am schnellsten und zu einem größtmöglichen Prozentsatz aus dem Zellplasma über die Mitochondrienmembran in die Mitochondrien.

Überraschender weise gelingt dies, wenn das erfindungsgemäß einzusetzende Peptid mindestens zwei Aminosäuren mit aromatischen Seitenketten aufweist, von denen mindestens eine Aminosäure ausgewählt ist aus Tyr, 2'-Methyltyrosin, 6'-Methyltyrosin, 2',6'-Dimethyltyrosin (Dmt), N,2',6'-Trimethyltyrosin (Tmt) oder 2'-Hydroxy-6'-Methyltyrosin (Hmt).

Gemäß einer besonders bevorzugten Ausführungsform der Erfindung enthält das Peptid mindestens eine D-Aminosäure. Dies hat den Vorteil, dass das Peptid von den haut- bzw. zelleigenen Abwehrprozessen, insbesondere den Aminopeptidasen, schlechter erkannt und nicht verdaut wird, bevor es zur Mitochondrienmembran in den Fibroblastenzellen gelangen kann. Insbesondere geeignet ist der Einsatz von D-Aminosäuren an der 1. und/oder 2. Stelle, vom N-Terminus aus gesehen.

Gemäß einer besonders bevorzugten Ausführungsform der Erfindung liegt die C-terminale Carboxylgruppe der Aminosäure am C-Terminus amidiert vor. Diese Amidierung verringert ebenfalls die Hydrolyse des Peptids vom C-Terminus aus.

Gemäß einer weiteren bevorzugten Ausführungsform der Erfindung beträgt das Verhältnis der Anzahl der Aminosäuren mit aromatischen Seitenketten zur Anzahl der positiven Nettoladungen des Peptids 0,3 bis 3, bevorzugt 0,6 bis 2. Die genannten Verhältnisse haben sich als optimal erwiesen, eine Passage durch die Zellmembranen, insbesondere die Mitochondrienmembran zu gewährleisten.

Gemäß einer besonders bevorzugten Ausführungsform der Erfindung umfasst das Peptid die Aminosäurenfolge Xxx-Lys, wobei die Aminosäure Xxx ausgewählt ist aus Phe, 2',6'-Dimethylphenylalanin (Dmp), Trp, Tyr, 2'-Methyltyrosin, 6'-Methyltyrosin, 2',6'-Dimethyltyrosin (Dmt), N,2',6'-Trimethyltyrosin (Tmt) oder 2'-Hydroxy-6'-Methyltyrosin (Hmt). Die Aminosäuresequenz Xxx-Lys kann dabei sowohl am N-Terminus, in der Mitte des Peptids als auch am C-Terminus auftreten.

Gemäß einer weiteren besonders bevorzugten Ausführungsform der Erfindung ist das Peptid ausgewählt aus Peptiden welche die folgenden Aminosäuresequenzen umfassen: (D-Arg)-Dmt-Lys-Phe-NH₂, Dmt-(D-Arg)-Phe-Lys-NH₂, Tyr-(D-Arg)-Phe-Lys-NH₂, Phe-(D-Arg)-Dmt-Lys-NH₂ und 2',6'-Dmp-(D-Arg)-Dmt-Lys-NH₂.

Umfassen bedeutet in diesem Zusammenhang, dass die Peptide die Sequenz zeigen und ggf. noch weitere Aminosäuren am N- und/oder C-Terminus tragen. Insbesondere sind solche Pepitde bevorzugt, die genau diese Aminosäuresequenz aufweisen (d.h. keine weiteren Aminosäuren am N- und/oder C-Terminus tragen).

Ganz besonders bevorzugt sind Peptide, die die Aminosäuresequenz (D-Arg)-Dmt-Lys-Phe-NH₂ und Dmt-(D-Arg)-Phe-Lys-NH₂ umfassen, insbesondere solche Peptide, die genau diese Sequenz aufweisen.

Gemäß einer besonders bevorzugten Ausführungsform der Erfindung enthalten die erfindungsgemäßen Zusammensetzung das mindestens eine Peptid in einer Konzentration von 0,00001 bis 10 Gew.-%, bevorzugt zu 0,0001 bis 1 Gew.-%, besonders bevorzugt 0,001 bis 0,5 Gew.-%, außerordentlich bevorzugt 0,005 - 0,1 Gew.-%, weiter bevorzugt 0,01 - 0,05 Gew.-%, jeweils bezogen auf das Gesamtgewicht der Zusammensetzung. Sind mehrere, insbesondere erfindungsgemäße Peptide enthalten, bezieht sich die angegebene Menge auf die Gesamtmenge an Peptid.

Gemäß einer bevorzugten Ausführungsform der Erfindung enthält die erfindungsgemäße Zusammensetzung, enthaltend ein antioxidatives Peptid und mindestens ein Tensid, mindestens einen zusätzlichen Wirkstoff, ausgewählt aus Aporphin-Alkaloiden, Purin und/oder Purinderivaten, natürlichen Betainverbindungen, alkyl- oder hydroxyalkylsubstituierten Harnstoffverbindungen, Monomeren, Oligomeren und Polymeren von Aminosäuren, N-C₂-C₂₄-Acylaminosäuren und/oder den Estern und/oder den physiologisch verträglichen Salzen dieser Substanzen, Polysacchariden, sowie Mischungen dieser Wirkstoffe. Überraschend wurde festgestellt, dass die Wirkung der erfindungsgemäßen Zusammensetzung in unerwarteter Weise durch ein Aporphin-Alkaloid gesteigert werden kann. Ohne an diese Theorie gebunden sein zu wollen, wird vermutet, dass der Zusatz von mindestens einem Aporphin-Alkaloid zu einer optimierten Regulierung der Elektrolytversorgung auf zellulärer Ebene führt, wodurch die Wirkung der erfindungsgemäßen Zusammensetzung bei der Bekämpfung der Hautalterung, zum Schutz der Haut vor Schäden durch ultraviolette Strahlung, zur Verbesserung der Elastizität der Haut und/oder zur Verringerung und/oder Verhinderung der Faltenbildung synergistisch verbessert wird.

Erfindungsgemäß bevorzugt zu verwendende Aporphin-Alkaloide sind ausgewählt ist aus Verbindungen der allgemeinen Struktur (APO-ALK-I), 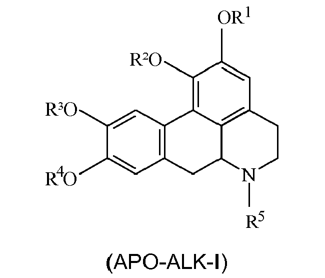 in der die Reste R¹, R², R³, R⁴ und R⁵, die gleich oder verschieden sein können, ausgewählt sind aus einem Wasserstoffatom, einem Halogenatom, einem C₁- bis C₄-Alkylrest, einem C₁- bis C₄-Monohydroxyalkylrest, einem C₂- bis C₄-Polyhydroxyalkylrest, einem (C₁- bis C₄)-Alkoxy-(C₁- bis C₄)-alkylrest, einem C₁- bis C₄-Aminoalkylrest, einem Hydroxy-(C₁- bis C₄)-alkylaminorest, einem C₁- bis C₄-Hydroxyalkoxyrest, einem C₁- bis C₄-Hydroxyalkyl-(C₁-bis C₄)-aminoalkylrest oder einem (Di-C₁- bis C₄-Alkylamino)-(C₁- bis C₄)-alkylrest, einer gegebenenfalls substituierten Arylgruppe, insbesondere einer Phenylgruppe, einer gegebenenfalls substituierten Heteroarylgruppe, einer Aryl-C₁-C₆-alkylgruppe, einer Acylgruppe, einer Sulfonylgruppe oder einem Zucker, und den kosmetisch verträglichen Salzen von (APO-ALK-I) mit einer Säure, in Form aller 15 optischen Isomere, in isomerenreiner Form oder als Mischung optischer Isomere.

Beispiele für die als Substituenten in den erfindungsgemäß kombinierten Verbindungen genannten C₁- bis C₄-Alkylreste sind die Gruppen Methyl, Ethyl, Propyl, Isopropyl und Butyl. Ethyl und Methyl sind bevorzugte Alkylreste. Erfindungsgemäß bevorzugt zu verwendende C₁- bis C₄-Alkoxyreste sind beispielsweise eine Methoxy- oder eine Ethoxygruppe. Weiterhin können als bevorzugte Beispiele für eine C₁- bis C₄-Hydroxyalkylgruppe eine Hydroxymethyl-, eine 2-Hydroxyethyl-, eine 3-Hydroxypropyl- oder eine 4-Hydroxybutylgruppe genannt werden. Eine 2-Hydroxyethylgruppe ist besonders bevorzugt. Eine besonders bevorzugte C₂- bis C₄-Polyhydroxyalkylgruppe ist die 1,2-Dihydroxyethylgruppe. Beispiele für Halogenatome sind erfindungsgemäß F-, Cl- oder Br-Atome, Cl-Atome sind ganz besonders bevorzugt.

Als Zuckerrest können beliebige Mono- oder Oligo-, insbesondere Disaccharide, eingesetzt werden. Üblicherweise werden Zucker mit 5 bzw. 6 Kohlenstoffatomen sowie die entsprechenden Oligosaccharide eingesetzt. Solche Zucker sind beispielsweise Glucose, Fructose, Galactose, Arabinose, Ribose, Xylose, Lyxose, Allose, Altrose, Mannose, Gulose, Idose, Talose und Sucrose. Bevorzugte Zuckerbausteine sind Glucose, Fructose, Galactose, Arabinose und Sucrose; Glucose ist besonders bevorzugt.

Besonders bevorzugte Aporphin-Alkaloide sind ausgewählt aus Verbindungen der allgemeinen Struktur (APO-ALK-I), in denen R¹ = R² = R³ = R⁴ = R⁵ = CH₃ ist. Diese Verbindung wird auch als 1,2,9,10-Tetramethoxyaporphin bezeichnet.

Besonders bevorzugt erfindungsgemäße Zusammensetzungen enthalten mindestens ein Aporphin-Alkaloid in engeren Mengenbereichen. Hier sind erfindungsgemäß bevorzugte Zusammensetzungen, insbesondere kosmetische Zusammensetzungen, **dadurch gekennzeichnet, dass** sie - bezogen auf ihr Gewicht - eine Gesamtmenge von 0,0001 - 1 Gew.-%, vorzugsweise 0,001 - 0,5 Gew.-%, besonders bevorzugt 0,002 - 0,1 Gew.-% und insbesondere 0,005 - 0,01 Gew.-% mindestens eines Aporphin-Alkaloids enthalten.

Erfindungsgemäß besonders bevorzugte Zusammensetzungen enthalten (D-Arg)-Dmt-Lys-Phe-NH₂ und 1,2,9,10-Tetramethoxyaporphin. Weitere erfindungsgemäß besonders bevorzugte Zusammensetzungen enthalten Dmt-(D-Arg)-Phe-Lys-NH₂ und 1,2,9,10-Tetramethoxyaporphin.

Ein weiterer Gegenstand der vorliegenden Erfindung ist eine Zusammensetzung, enthaltend ein antioxidatives Peptid gemäß Anspruch 1 und mindestens ein Tensid, in Kombination mit mindestens einem Wirkstoff enthalten, der ausgewählt ist aus Purin (7*H*-Imidazo[4,5-*d*]pyrimidin) und den Derivaten des Purins. Überraschend wurde festgestellt, dass die Wirkung der erfindungsgemäßen Zusammensetzung in unerwarteter Weise durch Purin und/oder Purinderivat(e) gesteigert werden kann. Ohne an diese Theorie gebinden sein zu wollen, wird vermutet, dass der Zusatz von Purin und/oder mindestens einem Purinderivat zu einer optimierten Regulierung der Nährstoffversorgung, insbesondere der Lipidversorgung, auf zellulärer Ebene führt, wodurch die Wirkung der erfindungsgemäßen Zusammensetzung bei der Bekämpfung der Hautalterung, zum Schutz der Haut vor Schäden durch ultraviolette Strahlung, zur Verbesserung der Elastizität der Haut und/oder zur Verringerung und/oder Verhinderung der Faltenbildung synergistisch verbessert wird.

Besonders bevorzugte erfindungsgemäße kosmetische oder dermatologische Zusammensetzungen enthalten Purin und/oder Purinderivat(e) - bezogen auf ihr Gewicht - in einer Gesamtmenge von 0,001 - 2,5 Gew.-%, bevorzugt 0,01 - 1 Gew.-%, besonders bevorzugt 0,1 - 0,8 Gew.-% und insbesondere 0,2 - 0,5 Gew.-%. Unter Purin, den Purinen und den Purinderivaten sind einige Vertreter erfindungsgemäß besonders bevorzugt. Erfindungsgemäß bevorzugte Zusammensetzungen enthalten Purin und/oder Purinderivat(e) der Formel (PUR-I) enthalten, 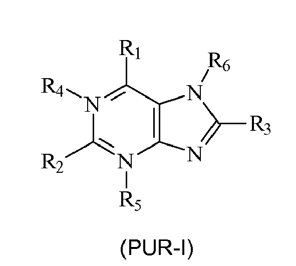 in der die Reste R¹, R² und R³ unabhängig voneinander ausgewählt sind aus -H, - OH, -NH₂, -SH und die Reste R⁴, R⁵ und R⁶ unabhängig voneinander ausgewählt sind aus -H, einem C₁- bis C₄-Alkylrest, einem C₁- bis C₄-Monohydroxyalkylrest, einem C₂- bis C₄-Polyhydroxyalkylrest, einem (C₁- bis C₄)-Alkoxy-(C₁- bis C₄)-alkylrest, einem C₁- bis C₄-Aminoalkylrest, einem Hydroxy-(C₁- bis C₄)-alkylaminorest, einem C₁- bis C₄-Hydroxyalkoxyrest, einem C₁- bis C₄-Hydroxyalkyl-(C₁- bis C₄)-aminoalkylrest oder einem (Di-C₁- bis C₄-Alkylamino)-(C₁- bis C₄)-alkylrest, einer gegebenenfalls substituierten Arylgruppe, insbesondere einer Phenylgruppe, einer gegebenenfalls substituierten Heteroarylgruppe und einer Aryl-C₁-C₆-alkylgruppe.

Erfindungsgemäß besonders bevorzugt zu verwendende Kombinationen sind **dadurch gekennzeichnet, dass** sie Purin und/oder Purinderivat(e) der Formel (PUR-I) enthalten, in der die Reste R¹, R² und R³ unabhängig voneinander ausgewählt sind aus -H, - OH, -NH₂, -SH und die Reste R⁴, R⁵ und R⁶ unabhängig voneinander ausgewählt sind aus -H, -CH₃ und -CH₂-CH₃, wobei folgende Verbindungen außerordentlich bevorzugt sind:
- Purin (R¹ = R² = R³ = R⁴ = R⁵ = R⁶ = H)
- Adenin (R¹ = NH₂, R² = R³ = R⁴ = R⁵ = R⁶ = H)
- Guanin (R¹ = OH, R² = NH₂, R³ = R⁴ = R⁵ = R⁶ = H)
- Harnsäure (R¹ = R² = R³ = OH, R⁴ = R⁵ = R⁶ = H)
- Hypoxanthin (R¹ = OH, R² = R³ = R⁴ = R⁵ = R⁶ = H)
- 6-Purinthiol (R¹ = SH, R² = R³ = R⁴ = R⁵ = R⁶ = H)
- 6-Thioguanin (R¹ = SH, R² = NH₂, R³ = R⁴ = R⁵ = R⁶ = H)
- Xanthin (R¹ = R² = OH, R³ = R⁴ = R⁵ = R⁶ = H)
- Coffein (R¹ = R² = OH, R³ = H, R⁴ = R⁵ = R⁶ = CH₃)
- Theobromin (R¹ = R² = OH, R³ = R⁴ = H, R⁵ = R⁶ = CH₃)
- Theophyllin (R¹ = R² = OH, R³ = H, R⁴ = CH₃, R⁵ = CH₃, R⁶ = H).

Verbindungen der allgemeinen Formel (PUR-I), in denen R¹ und/oder R² einer OH-Gruppe entsprechen, existieren in tautomeren Verbindungen, insbesondere solchen der allgemeinen Formel (PUR-II), ausgehend von (PUR-I) mit R¹ = R² = OH: 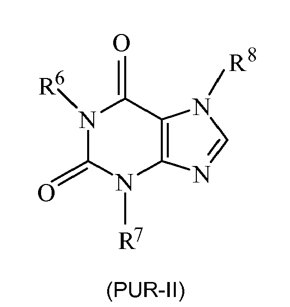 wobei in der Formel (PUR-II) die Reste R⁶, R⁷ und R⁸, die gleich oder verschieden sein können, ausgewählt sind aus einem Wasserstoffatom, einem C₁- bis C₄-Alkylrest, einem C₁- bis C₄-Mono-hydroxyalkylrest, einem C₂- bis C₄-Polyhydroxyalkylrest, einem (C₁- bis C₄)-Alkoxy-(C₁- bis C₄)-alkylrest, einem C₁- bis C₄-Aminoalkylrest, einem Hydroxy-(C₁- bis C₄)-alkylaminorest, einem C₁- bis C₄-Hydroxyalkoxyrest, einem C₁- bis C₄-Hydroxyalkyl-(C₁-bis C₄)-aminoalkylrest oder einem (Di-C₁- bis C₄-Alkylamino)-(C₁- bis C₄)-alkylrest, einer gegebenenfalls substituierten Arylgruppe, insbesondere einer Phenylgruppe, einer gegebenenfalls substituierten Heteroarylgruppe und einer Aryl-C₁-C₆-alkylgruppe, wobei Coffein (R⁶ = R⁷ = R⁸ = CH₃), Theobromin (R⁶ = H, R⁷ = R⁸ = CH₃) und Theophyllin (R⁶ = R⁷ = CH₃, R⁸ = H) außerordentlich bevorzugt sind.

Erfindungsgemäß besonders bevorzugte Zusammensetzungen enthalten (D-Arg)-Dmt-Lys-Phe-NH₂ und Coffein. Weitere erfindungsgemäß besonders bevorzugte Zusammensetzungen enthalten (D-Arg)-Dmt-Lys-Phe-NH₂ und Theobromin. Weitere erfindungsgemäß besonders bevorzugte Zusammensetzungen enthalten (D-Arg)-Dmt-Lys-Phe-NH₂ und Theophyllin. Weitere erfindungsgemäß besonders bevorzugte Zusammensetzungen enthalten Dmt-(D-Arg)-Phe-Lys-NH₂ und

Coffein. Weitere erfindungsgemäß besonders bevorzugte Zusammensetzungen sind gekennzeichnet durch einen Gehalt an Dmt-(D-Arg)-Phe-Lys-NH₂ und Theobromin. Weitere erfindungsgemäß besonders bevorzugte Zusammensetzungen sind gekennzeichnet durch einen Gehalt an Dmt-(D-Arg)-Phe-Lys-NH₂ und Theophyllin.

Ein weiterer Gegenstand der vorliegenden Erfindung ist eine Zusammensetzung, enthaltend ein antioxidatives Peptid gemäß Anspruch 1 und mindestens ein Tensid, in Kombination mit mindestens einer natürlichen Betainverbindung.

Überraschend wurde festgestellt, dass die Wirkung der erfindungsgemäßen Zusammensetzung in unerwarteter Weise durch eine natürliche Betainverbindung gesteigert werden kann. Ohne an diese Theorie gebunden sein zu wollen, wird vermutet, dass der Zusatz von mindestens einer natürlichen Betainverbindung zu einer optimierten Regulierung der Nährstoffversorgung auf zellulärer Ebene und/oder innerhalb der extracellulären Matrix (ECM) führt, wodurch die Wirkung der erfindungsgemäßen Zusammensetzung bei der Bekämpfung der Hautalterung, zum Schutz der Haut vor Schäden durch ultraviolette Strahlung, zur Verbesserung der Elastizität der Haut und/oder zur Verringerung und/oder Verhinderung der Faltenbildung synergistisch verbessert wird.

Erfindungsgemäß bevorzugt zu verwendende natürliche Betainverbindungen sind natürlich vorkommende Verbindungen mit der Atomgruppierung R₃N⁺-CH₂-X-COO⁻ gemäß IUPAC-Regel C-816.1. Sogenannte Betaintenside (synthetisch) fallen nicht unter die erfindungsgemäß verwendeten Betainverbindungen, ebenso wenig andere zwitterionische Verbindungen, in denen sich die positive Ladung an N oder P und die negative Ladung formal an O, S, B oder C befindet, die aber nicht der IUPAC-Regel C-816.1 entsprechen. Erfindungsgemäß bevorzugte Betainverbindungen sind Betain (Me₃N⁺-CH₂-COO⁻) Carnitin (Me₃N⁺-CH₂-CHOH-CH₂-COO⁻), jeweils mit Me = Methyl, und Ergothionein = 2-Mercapto-N^{α}N^{α}N^{α}-trimethyl-L-histidinium-Betain der Formel (BETA-II)

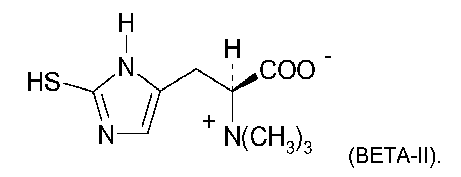

Die Herkunft der eingesetzten natürlichen Betainverbindung kann erfindungsgemäß durchaus synthetisch sein. Weiterhin können auch Salze und/oder Ester der natürlichen Betainverbindungen erfindungsgemäß bevorzugt eingesetzt werden. Ein derartiges besonders bevorzugtes Derivat ist Carnitintartrat. Bevorzugte erfindungsgemäße Zusammensetzungen enthalten zusätzlich mindestens eine natürliche Betainverbindung in einer Gesamtmenge von 0,01 bis 5 Gew.-%, bevorzugt 0,1 bis 3 Gew.-%, besonders bevorzugt 0,2 bis 1 Gew.-% und außerordentlich bevorzugt 0,3 - 0,5 Gew.-%, jeweils bezogen auf die gesamte Zusammensetzung.

Erfindungsgemäß besonders bevorzugte Zusammensetzungen enthalten Betain und ein Peptid, ausgewählt aus (D-Arg)-Dmt-Lys-Phe-NH₂ und Dmt-(D-Arg)-Phe-Lys-NH₂. Andere ebenfalls erfindungsgemäß besonders bevorzugte Kombinationen sind Carnitin und (D-Arg)-Dmt-Lys-Phe-NH₂ oder Dmt-(D-Arg)-Phe-Lys-NH₂, ebenso Carnitintartrat und ein antioxidatives Peptid, ausgewählt aus (D-Arg)-Dmt-Lys-Phe-NH₂ und Dmt-(D-Arg)-Phe-Lys-NH₂. Weitere erfindungsgemäß besonders bevorzugte Zusammensetzungen enthalten Ergothionein und ein antioxidatives Peptid, ausgewählt aus (D-Arg)-Dmt-Lys-Phe-NH₂ oder Dmt-(D-Arg)-Phe-Lys-NH₂.

Ein weiterer Gegenstand der vorliegenden Erfindung ist eine Zusammensetzung, enthaltend ein antioxidatives Peptid gemäß Anspruch 1 und mindestens ein Tensid, in Kombination mit mindestens einer Substanz, die ausgewählt ist aus Harnstoff und alkyl- oder hydroxyalkylsubstituierten Harnstoffen. Überraschend wurde festgestellt, dass die Wirkung der erfindungsgemäßen Zusammensetzung in unerwarteter Weise durch Substanzen, ausgewählt aus Harnstoff und alkyl- oder hydroxyalkylsubstituierten Harnstoffen, gesteigert werden kann. Ohne an diese Theorie gebunden sein zu wollen, wird vermutet, dass der Zusatz von mindestens einer Substanz, ausgewählt aus Harnstoff und alkyl- oder hydroxyalkylsubstituierten Harnstoffen, zu einer optimierten Regulierung der Nährstoffversorgung innerhalb der extracellulären Matrix (ECM) führt, wodurch die Wirkung der erfindungsgemäßen Zusammensetzung bei der Bekämpfung der Hautalterung, zum Schutz der Haut vor Schäden durch ultraviolette Strahlung, zur Verbesserung der Elastizität der Haut und/oder zur Verringerung und/oder Verhinderung der Faltenbildung synergistisch verbessert wird. Erfindungsgemäß bevorzugt zu verwendende alkyl- oder hydroxyalkylsubstituierte Harnstoffverbindungen weisen die allgemeine Formel (UREA-I) auf, 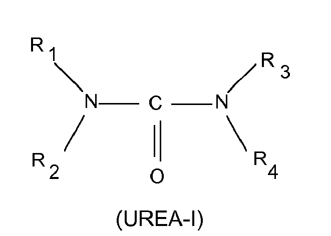 in der R₁, R₂, R₃ und R₄ unabhängig voneinander für ein Wasserstoffatom, eine Methyl-, Ethyl-, n-Propyl-, Isopropyl-, n-Butyl-, sek.-Butyl-, tert. Butyl- oder C₂-C₆-Hydroxyalkyl-Gruppe, die mit 1 bis 5 Hydroxylgruppen oder C₁-C₄-Hydroxyalkyl-Gruppen substituiert ist, stehen, mit der Maßgabe, dass mindestens einer der Reste R₁ - R₄ eine C₂-C₆-Hydroxyalkyl-Gruppe, die mit 1 bis 5 Hydroxylgruppen oder C₁-C₄-Hydroxyalkyl-Gruppen substituiert ist, darstellt.

Erfindungsgemäß besonders bevorzugte alkyl- oder hydroxyalkylsubstituierte Harnstoffverbindungen der allgemeinen Formel (UREA-I) sind ausgewählt aus Verbindungen, bei denen jeweils mindestens einer der Reste R₁ und R₂ bzw. R₃ und R₄ eine C₂-C₆-Hydroxyalkyl-Gruppe, die mit 1 bis 5 Hydroxylgruppen oder C₁-C₄-Hydroxyalkyl-Gruppen substituiert ist, darstellt. Besonders bevorzugte C₂-C₆-Hydroxyalkyl-Gruppen, die mit 1 bis 5 Hydroxylgruppen oder C₁-C₄-Hydroxyalkyl-Gruppen substituiert sind, sind ausgewählt aus 2-Hydroxyethyl-, 3-Hydroxypropyl-, 2-Hydroxypropyl, 2-Hydroxyisopropyl- und 4-Hydroxybutyl-Gruppen, wobei die 2-Hydroxyethyl-Gruppe außerordentlich bevorzugt ist. Ebenfalls außerordentlich bevorzugt ist Bis-N,N'-(2-Hydroxyethyl)-harnstoff. Harnstoff selbst ist ebenfalls besonders bevorzugt. Weiterhin bevorzugt ist es, Mischungen von Harnstoff und alkyl- oder hydroxyalkylsubstituierten Harnstoffverbindungen einzusetzen. Die handelsüblich erhältlichen alkyl- oder hydroxyalkylsubstituierten Harnstoff-Zubereitungen enthalten meistens als Neben- oder Abbauprodukt unsubstituierten Harnstoff. Daher sind erfindungsgemäß auch Kombinationen aus Harnstoff und mindestens einer Substanz, ausgewählt aus alkyl- oder hydroxyalkylsubstituierten Harnstoffen, bevorzugt. Außerordentlich bevorzugt sind Mischungen von Harnstoff und Bis-N,N'-(2-Hydroxyethyl)-harnstoff. Bevorzugte erfindungsgemäß einzusetzende Zusammensetzungen enthalten mindestens eine Substanz, ausgewählt aus Harnstoff und alkyl-oder hydroxyalkylsubstituierten Harnstoffen, in einer Gesamtmenge von 0,01 bis 10 Gew.-%, bevorzugt 0,1 bis 5 Gew.-%, besonders bevorzugt 0,2 bis 3 Gew.-% und außerordentlich bevorzugt 0,5 - 1 Gew.-%, jeweils bezogen auf die gesamte Zusammensetzung.

Die handelsüblich erhältlichen alkyl- oder hydroxyalkylsubstituierten Harnstoff-Zubereitungen enthalten meistens als Neben- oder Abbauprodukt unsubstituierten Harnstoff. Daher sind erfindungsgemäß auch Kombinationen aus Harnstoff und mindestens einer Substanz, ausgewählt aus alkyl- oder hydroxyalkylsubstituierten Harnstoffen, bevorzugt.

Erfindungsgemäß besonders bevorzugte Kombinationen sind Bis-N,N'-(2-Hydroxyethyl)-harnstoff und ein antioxidatives Peptid, ausgewählt aus (D-Arg)-Dmt-Lys-Phe-NH₂ und Dmt-(D-Arg)-Phe-Lys-NH₂. Weitere erfindungsgemäß besonders bevorzugte Zusammensetzungen enthalten Harnstoff und ein Peptid, ausgewählt aus (D-Arg)-Dmt-Lys-Phe-NH₂ und Dmt-(D-Arg)-Phe-Lys-NH₂. Weitere erfindungsgemäß besonders bevorzugte Zusammensetzungen enthalten Harnstoff und Bis-N,N'-(2-Hydroxyethyl)-harnstoff sowie ein Peptid, ausgewählt aus (D-Arg)-Dmt-Lys-Phe-NH₂ und Dmt-(D-Arg)-Phe-Lys-NH₂.

Ein weiterer Gegenstand der vorliegenden Erfindung ist eine Zusammensetzung, enthaltend eine antioxidative Substanz und mindestens ein Tensid, in Kombination mit mindestens einer Substanz, die ausgewählt ist aus Monomeren, Oligomeren und Polymeren von Aminosäuren, N-C₂-C₂₄-Acyl-aminosäuren und/oder den Estern und/oder den physiologisch verträglichen Salzen dieser Substanzen, sowie Mischungen dieser Wirkstoffe.

Überraschend wurde festgestellt, dass die Wirkung der erfindungsgemäßen Zusammensetzungin unerwarteter Weise durch Substanzen, ausgewählt aus Monomeren, Oligomeren und Polymeren von Aminosäuren, N-C₂-C₂₄-Acylaminosäuren und/oder den Estern und/oder den physiologisch verträglichen Salzen dieser Substanzen, sowie Mischungen dieser Wirkstoffe, gesteigert werden kann. Ohne an diese Theorie gebunden sein zu wollen, wird vermutet, dass der Zusatz von mindestens einer Substanz, ausgewählt aus Monomeren, Oligomeren und Polymeren von Aminosäuren, N-C₂-C₂₄-Acylaminosäuren und/oder den Estern und/oder den physiologisch verträglichen Salzen dieser Substanzen, sowie Mischungen dieser Wirkstoffe, zu einer optimierten Regulierung der Nährstoffversorgung auf zellulärer Ebene und/oder zu einer optimierten Regulierung der Nährstoffversorgung innerhalb der extracellulären Matrix (ECM) und/oder zu einer Stimulierung der Aufbauprozesse der extracellulären Matrix (ECM) führt, wodurch wodurch die Wirkung der erfindungsgemäßen Zusammensetzung bei der Bekämpfung der Hautalterung, zum Schutz der Haut vor Schäden durch ultraviolette Strahlung, zur Verbesserung der Elastizität der Haut und/oder zur Verringerung und/oder Verhinderung der Faltenbildung synergistisch verbessert wird.

Die Monomere der Aminosäuren und/oder der N-C₂-C₂₄-Acylaminosäuren sind bevorzugt ausgewählt aus Alanin, Arginin, Asparagin, Asparaginsäure, Canavanin, Citrullin, Cystein, Cystin, Dipalmitoylhydroxyprolin, Desmosin, Glutamin, Glutaminsäure, Glycin, Histidin, Homophenylalanin, Hydroxylysin, Hydroxyprolin, Isodesmosin, Isoleucin, Leucin, Lysin, Methionin, Methylnorleucin, Ornithin, Phenylalanin, Prolin, Pyroglutaminsäure, Sarcosin, Serin, Taurin, Threonin, Thyroxin, Tryptophan, Tyrosin, Valin, N-Acetyl-L-cystein, Zinkpyroglutamat, Natriumoctanoylglutamat, Natriumdecanoylglutamat, Natriumlauroylglutamat, Natriummyristoylglutamat, Natriumcetoylglutamat und Natriumstearoylglutamat. Besonders bevorzugt sind Lysin, Serin, Zink- und Natriumpyroglutamat und Natriumlauroylglutamat.

Der C₂ - C₂₄-Acylrest, mit dem die genannten Aminosäuren an der Aminogruppe derivatisiert sind, ist bevorzugt ausgewählt aus einem Acetyl-, Propanoyl-, Butanoyl-, Pentanoyl-, Hexanoyl-, Heptanoyl-, Octanoyl-, Nonanoyl-, Decanoyl-, Undecanoyl-, Lauroyl-, Tridecanoyl-, Myristoyl-, Pentadecanoyl-, Cetoyl-, Palmitoyl-, Stearoyl-, Elaidoyl-, Arachidoyl- oder Behenoyl-Rest. Mischungen von C₈-C₁₈-Acylresten werden auch als Cocoyl-Rest bezeichnet und sind ebenfalls bevorzugte Substituenten. Mit den vorgenannten C₂ - C₂₄-Acylresten können die Aminosäuren, die eine OH-Gruppe tragen, auch an dieser OH-Gruppe verestert sein. Ein erfindungsgemäß bevorzugtes Beispiel hierfür ist Hydroxyprolin, das mit zwei, bevorzugt linearen, C₂-C₂₂-Fettsäureresten N-acyliert und verestert ist, besonders bevorzugt Dipalmitoylhydroxyprolin.

Die physiologisch verträglichen Salze der erfindungsgemäß bevorzugten Aminosäuren oder Aminosäurederivate sind bevorzugt ausgewählt aus den Ammonium-, Alkalimetall-, Magnesium-, Calcium-, Aluminium-, Zink- und Mangan-Salzen. Besonders bevorzugt sind die Natrium-, Kalium-, Magnesium-, Aluminium-, Zink- und Mangan-Salze.

Unter Aminosäureoligomeren werden erfindungsgemäß Peptide mit 2 - 30, bevorzugt 2 - 15, Aminosäuren, verstanden. Die Oligomere der Aminosäuren und/oder der N-C₂-C₂₄-Acylaminosäuren sind bevorzugt ausgewählt aus Di-, Tri-, Tetra-, Penta-, Hexa- oder Pentadecapeptiden, die N-acyliert und/oder verestert sein können. Zahlreiche dieser Aminosäureoligomere stimulieren die Collagensynthese beziehungsweise sind in der Lage, Zellen des Immunsystems, wie Mastzellen und Makrophagen, zu rekrutieren, die dann über die Freisetzung von Wachstumsfaktoren Reparaturprozesse im Gewebe, z.B. die Collagensynthese, induzieren, beziehungsweise sind in der Lage, an die Sequenz Arg-Phe-Lys in Thrombospondin I (TSP-1) zu binden und damit aktives TGF-β (*tissue growth factor*), der die Synthese von Collagen in dermalen Fibroblasten induziert, freizusetzen.

Erfindungsgemäß bevorzugte, gegebenenfalls N-acylierte und/oder veresterte Dipeptide sind Acetyl-Citrullyl-Arginin (INCl:Acetyl Citrull Amido Arginine), Tyr-Arg (Dipeptide-1), Val-Trp (Dipeptide-2), Asn-Phe, Asp-Phe, N-Palmitoyl-β-Ala-His, N-Acetyl-Tyr-Arg-hexyldecylester, Carnosin (β-Ala-His) und N-Palmitoyl-Pro-Arg. Erfindungsgemäß bevorzugte, gegebenenfalls N-acylierte und/oder veresterte Tripeptide sind Gly-His-Lys und N-Palmitoyl-Gly-His-Lys. Weiterhin können Analoga von Gly-His-Lys eingesetzt werden, wobei maximal zwei Aminosäuren durch geeignete andere Aminosäuren substituiert sind. Zur Substitution von Gly sind erfindungsgemäß Ala, Leu und

Ile geeignet. Die erfindungsgemäß bevorzugten Aminosäuren, die His oder Lys ersetzen können, beinhalten eine Seitenkette mit einem Stickstoffatom, das bei pH 6 überwiegend geladen vorliegt, z. B. Pro, Lys, Arg, His, Desmosin und Isodesmosin. Besonders bevorzugt wird Lys durch Arg, Orn, oder Citrullin ersetzt. Ein weiteres erfindungsgemäß bevorzugtes Tripeptid ist Gly-His-Arg (INCl-Bezeichnung: Tripeptide-3) sowie dessen Derivat N-Myristoyl-Gly-His-Arg; weitere erfindungsgemäß bevorzugt zu verwendende Tripeptide sind ausgewählt aus Lys-Val-Lys, Lys-Val-Dab (Dab = Diaminobuttersäure), Lys-Phe-Lys, Lys-Ile-Lys, Dab-Val-Lys, Lys-Val-Orn, Lys-Val-Dap (Dap = Diaminopropionsäure), Dap-Val-Lys, Palmitoyl-Lys-Val-Lys, Lys-Pro-Val, Tyr-Tyr-Val, Tyr-Val-Tyr, Val-Tyr-Val (Tripeptide-2), Tripeptide-4, His-Ala-Orn, Gly-Asp-Ser, N-Elaidoyl-Lys-Phe-Lys und N-Acetyl-Arg-Lys-Arg-NH₂.

Erfindungsgemäß bevorzugte, gegebenenfalls N-acylierte und/oder veresterte Tetrapeptide sind ausgewählt aus Rigin und Rigin-basierten Tetrapeptiden sowie ALAMCAT-Tetrapeptiden. Rigin weist die Sequenz Gly-Gln-Pro-Arg auf. Rigin-basierte Tetrapeptide umfassen die Rigin-Analoga und Rigin-Derivate, insbesondere das erfindungsgemäß besonders bevorzugte N-Palmitoyl-Gly-Gln-Pro-Arg. Zu den Rigin-Analoga zählen solche, bei denen die vier Aminosäuren umarrangiert sind und/oder bei denen gegenüber Rigin maximal zwei Aminosäuren substituiert sind, z. B. die Sequenz Ala-Gln-Thr-Arg. Bevorzugt hat mindestens eine der Aminosäuren der Sequenz ein Pro oder Arg und besonders bevorzugt beinhaltet das Tetrapeptid sowohl Pro als auch Arg, wobei ihre Reihenfolge und Position variieren können. Die substituierenden Aminosäuren können aus jeder Aminosäure, die im Folgenden definiert ist, ausgewählt werden. Besonders bevorzugte Rigin-basierte Tetrapetide umfassen: Xaa-Xbb-Arg-Xcc, Xaa-Xbb-Xcc-Pro, Xaa-Xbb-Pro-Arg, Xaa-Xbb-Pro-Xcc, Xaa-Xbb-Xcc-Arg, wobei Xaa, Xbb und Xcc gleiche oder voneinander verschiedene Aminosäuren sein können und wobei Xaa ausgewählt ist aus Gly und den Aminosäuren, die Gly substituieren können, Xbb ausgewählt ist aus Gln und den Aminosäuren, die Gln substituieren können, Xcc ausgewählt ist aus Pro oder Arg und den Aminosäuren, die Pro und Arg substituieren können. Die bevorzugten Aminosäuren, die Gly ersetzen können, beinhalten eine aliphatische Seitenkette, z. B. β-Ala, Ala, Val, Leu, Pro, Sarcosin (Sar) und Isoleucin (Ile). Die bevorzugten Aminosäuren, die Gln ersetzen können, beinhalten eine Seitenkette mit einer Aminogruppe, die bei neutralem pH (pH 6-7) überwiegend ungeladen vorliegt, z.B. Asn, Lys, Orn, 5-Hydroxyprolin, Citrullin und Canavanin. Die bevorzugten Aminosäuren, die Arg ersetzen können, beinhalten eine Seitenkette mit einem Stickstoffatom, das bei pH 6 überwiegend geladen vorliegt, z.B. Pro, Lys, His, Desmosin und Isodesmosin. Als Rigin-Analoga sind erfindungsgemäß Gly-Gln-Arg-Pro und Val-Val-Arg-Pro bevorzugt.

ALAMCAT-Tetrapeptide sind Tetrapeptide, die mindestens eine Aminosäure mit einer aliphatischen Seitenkette enthalten, z. B. β-Ala, Ala, Val, Leu, Pro, Sarcosin (Sar) und Isoleucin (Ile). Weiterhin beinhalten ALAMCAT-Tetrapeptide mindestens eine Aminosäure mit einer Seitenkette mit einer Aminogruppe, die bei neutralem pH (pH 6-7) überwiegend ungeladen vorliegt, z.B. Gln, Asn, Lys, Orn, 5-Hydroxyprolin, Citrullin und Canavanin. Weiterhin beinhalten ALAMCAT-Tetrapeptide mindestens eine Aminosäure mit einer Seitenkette mit einem Stickstoffatom, das bei pH 6 überwiegend geladen vorliegt, z. B. Arg, Pro, Lys, His, Desmosin und Isodesmosin. Als vierte Aminosäure können ALAMCAT-Tetrapeptide jede beliebige Aminosäure enthalten; bevorzugt ist jedoch auch die vierte Aminosäure aus den drei vorstehend genannten Gruppen ausgewählt.

Erfindungsgemäß bevorzugte, gegebenenfalls N-acylierte und/oder veresterte Pentapeptide sind ausgewählt aus Lys-Thr-Thr-Lys-Ser und seinen N-acylierten Derivaten, besonders bevorzugt N-Palmitoyl-Lys-Thr-Thr-Lys-Ser, weiterhin N-Palmitoyl-Tyr-Gly-Gly-Phe-Met, Val-Val-Arg-Pro-Pro, N-Palmitoyl-Tyr-Gly-Gly-Phe-Leu, Gly-Pro-Phe-Pro-Leu und N-Benzyloxycarbonyl-Gly-Pro-Phe-Pro-Leu (die beiden letztgenannten stellen Serinproteinase-Inhibitoren zur Inhibition der Desquamation dar). Erfindungsgemäß bevorzugte, gegebenenfalls N-acylierte und/oder veresterte Hexapeptide sind Val-Gly-Val-Ala-Pro-Gly und seine N-acylierten Derivate, besonders bevorzugt N-Palmitoyl-Val-Gly-Val-Ala-Pro-Gly, weiterhin Glu-Glu-Met-Gln-Arg-Arg, weiterhin Acetyl-Hexapeptide-3, Hexapeptide-4, Hexapeptide-5, Myristoyl Hexapeptide-5, Myristoyl Hexapeptide-6, Hexapeptide-8, Myristoyl Hexapeptide-8, Hexapeptide-9 und Hexapeptide-10, Ala-Arg-His-Leu-Phe-Trp (Hexapeptide-1), Acetyl Hexapeptide-1, Acetyl Glutamyl Hexapeptide-1, Hexapeptide-2, Ala-Asp-Leu-Lys-Pro-Thr (Hexapeptide-3), Val-Val-Arg-Pro-Pro-Pro, Hexapeptide-4, Hexapeptide-5, Myristoyl Hexapeptide-5, Myristoyl Hexapeptide-6, Ala-Arg-His-Methylnorleucin-Homophenylalanin-Trp (Hexapeptide-7), Hexapeptide-8, Myristoyl Hexapeptide-8, Hexapeptide-9, Hexapeptide-10 und Hexapeptide-11. Ein erfindungsgemäß bevorzugtes Pentadecapeptid ist z. B. Pentadecapeptide-1.

Ein weiteres bevorzugtes Aminosäureoligomer ist das Peptidderivat L-Glutamylaminoethyl-indol. Ein erfindungsgemäß besonders bevorzugter Aminosäureoligomer-Wirkstoff ist die Kombination aus N-Palmitoyl-Gly-His-Lys und N-Palmitoyl-Gly-Gln-Pro-Arg.

Ein weiterer erfindungsgemäß bevorzugter Wirkstoff, der in der erfindungsgemäßen Zusammensetzung eine unerwartete Wirkungssteigerung zeigt, ist ausgewählt aus Polymeren der Aminosäuren und/oder der N-C₂-C₂₄-Acylaminosäuren.

Erfindungsgemäß kombinierte Polymere der Aminosäuren und/oder der N-C₂-C₂₄-Acylaminosäuren sind bevorzugt ausgewählt aus pflanzlichen und tierischen Proteinhydrolysaten und/oder Proteinen. Tierische Proteinhydrolysate sind z. B. Elastin-, Collagen-, Keratin-, Seiden-, Conchiolin- und Milcheiweiß-Proteinhydrolysate, die auch in Form von Salzen vorliegen können. Erfindungsgemäß bevorzugt sind pflanzliche Proteinhydrolysate, z. B. Soja-, Weizen-, Mandel-, Erbsen-, Kartoffel-und Reisproteinhydrolysate. Besonders bevorzugt sind Sojaproteinhydrolysate, besonders bevorzugt Sojaproteinhydrolysate mit einem mittleren Molekulargewicht im Bereich von 1200 - 1800 Dalton, bevorzugt im Bereich von 1400 - 1700 Dalton, z. B. unter dem Handelsnamen Ridulisse C^{®} von der Firma Silab erhältlich, und Sojaproteinhydrolysate mit einem mittleren Molekulargewicht im Bereich von 600 - 1000 Dalton, bevorzugt 800 Dalton, z. B. unter dem Handelsnamen Phytokine^{®} von Coletica erhältlich. Ebenfalls bevorzugt ist der Einsatz von Acylderivaten der Proteinhydrolysate, z. B. in Form ihrer Fettsäure-Kondensationsprodukte. Erfindungsgemäß bevorzugt sind auch kationisierte Proteinhydrolysate.

In einer weiteren bevorzugten Ausführungsform sind die erfindungsgemäß kombinierten Polymeren der Aminosäuren ausgewählt aus DNA-Reparaturenzymen.

Erfindungsgemäß bevorzugt zu verwendende DNA-Reparaturenzyme sind Photolyase und T4 Endonuclease V, letztere im Weiteren mit "T4N5" abgekürzt. Diese beiden Enzyme sind im Stand der Technik bereits als sogenannte DNA-Reparatur-Enzyme bekannt. Unter DNA-Reparatur ist definitionsgemäß die Spaltung bzw. Entfernung von UV-induzierten Pyrimidindimeren aus der DNA zu verstehen. Erfindungsgemäß besonders bevorzugt ist der Einsatz von liposomenverkapselten DNA-Reparaturenzymen. Liposomenverkapselte Photolyase ist im Handel z. B. unter der Produktbezeichnung Photosome™, liposomenverkapselte T4N5 z. B. unter der Bezeichnung Ultrasome™ von der Firma AGI Dermatics, USA, erhältlich. Erfindungsgemäß besonders bevorzugte Zusammensetzungen enthalten zusätzlich mindestens eines der Handelsprodukte Photosomes™ oder Ultrasomes™ in Gesamtmengen von 0,1 - 10 Gew.-%, bevorzugt 0,5 - 5,0 Gew.-% und besonders bevorzugt 1,0 - 4,0 Gew.-%, bezogen auf die gesamte erfindungsgemäße Zusammensetzung. Weitere erfindungsgemäß besonders bevorzugte Zusammensetzungen enthalten das Enzym Photolyase in einer Gesamtmenge von 0,0001 - 0,1 Gew.-%, bevorzugt 0,001 - 0,05 Gew.-%, besonders bevorzugt 0,002 - 0,02 Gew.-% und außerordentlich bevorzugt 0,005 - 0,01 Gew.-%, jeweils bezogen auf die gesamte erfindungsgemäße Zusammensetzung.

Weitere erfindungsgemäß besonders bevorzugte Zusammensetzungen enthalten das Enzym T4 Endonuclease V in einer Gesamtmenge von 0,0001 - 0,1 Gew.-%, bevorzugt 0,001 - 0,05 Gew.-%, besonders bevorzugt 0,002 - 0,02 Gew.-% und außerordentlich bevorzugt 0,005 - 0,01 Gew.-%, jeweils bezogen auf die gesamte erfindungsgemäße Zusammensetzung.

Erfindungsgemäß besonders bevorzugte Zusammensetzungen enthalten Photolyase und ein Peptid, ausgewählt aus (D-Arg)-Dmt-Lys-Phe-NH₂ und Dmt-(D-Arg)-Phe-Lys-NH₂. Weitere erfindungsgemäß besonders bevorzugte Zusammensetzungen enthalten T4 Endonuclease V und ein Peptid, ausgewählt aus (D-Arg)-Dmt-Lys-Phe-NH₂ und Dmt-(D-Arg)-Phe-Lys-NH₂. Weitere erfindungsgemäß besonders bevorzugte Zusammensetzungen enthalten Photolyase und T4 Endonuclease V und ein Peptid, ausgewählt aus (D-Arg)-Dmt-Lys-Phe-NH₂ und Dmt-(D-Arg)-Phe-Lys-NH₂.

Erfindungsgemäß besonders bevorzugte Zusammensetzungen sind **dadurch gekennzeichnet, dass** sie zusätzlich mindestens einen Wirkstoff, der ausgewählt ist aus Monomeren, Oligomeren und

Polymeren von Aminosäuren, N-C₂-C₂₄-Acylaminosäuren und/oder den Estern und/oder den physiologisch verträglichen Salzen dieser Substanzen, in einer Gesamtmenge von 0,00001 - 2 Gew.-%, bevorzugt 0,0001 - 1 Gew.-%, besonders bevorzugt 0,001 - 0,1 Gew.-% und außerordentlich bevorzugt 0,01 - 0,05 Gew.-%, jeweils bezogen auf den Gehalt an Aktivsubstanz in der gesamten Zusammensetzung, enthalten.

Erfindungsgemäß besonders bevorzugte Zusammensetzungen enthalten ein antioxidatives Peptid, ausgewählt aus (D-Arg)-Dmt-Lys-Phe-NH₂ und Dmt-(D-Arg)-Phe-Lys-NH₂, und und mindestens ein Peptid, ausgewählt aus N-Palmitoyl-Gly-His-Lys und/oder N-Palmitoyl-Gly-Gln-Pro-Arg und/oder N-Palmitoyl-Lys-Thr-Thr-Lys-Ser.

Es kann erfindungsgemäß besonders bevorzugt sein, dass die als Wirkstoff eingesetzten Aminosäuren und Peptide zur Verbesserung der Penetration in die Haut mit mindestens einer, bevorzugt linearen, C₂-C₂₂-Fettsäure N-acyliert und/oder verestert sind. Zur N-Acylierung und/oder Veresterung besonders bevorzugt sind C₈-C₁₈-Fettsäuren, ganz besonders bevorzugt sind Myristinsäure (C₁₄) und Palmitinsäure (C₁₆). Besonders bevorzugt ist weiterhin, dass alle verwendeten Aminosäuren und Peptide derartig N-acyliert und/oder verestert sind. Ebenfalls bevorzugt ist die Substitution der Aminosäuren und Peptide mit einer Benzyloxycarbonylgruppe an der terminalen Aminogruppe.

Ein weiterer erfindungsgemäß bevorzugter Wirkstoff, der in Gegenwart der erfindungsgemäßen Zusammensetzung, eine unerwartete Wirkungssteigerung zeigt, ist ausgewählt aus Polysacchariden. Überraschend wurde festgestellt, dass die Wirkung der erfindungsgemäßen Zusammensetzung, in unerwarteter Weise durch Polysaccharide, insbesondere durch Polysaccharide, die mindestens einen Desoxyzucker-Baustein, insbesondere Fucose und/oder Rhamnose, enthalten, und/oder durch Glycosaminoglycane und/oder durch Glucane gesteigert werden kann. Ohne an diese Theorie gebunden sein zu wollen, wird vermutet, dass der Zusatz von mindestens einem Polysaccharid zu einer optimierten Regulierung des Wasserhaushalts innerhalb der extracellulären Matrix (ECM) und/oder zu einer optimierten Regulierung der Nährstoffversorgung innerhalb der ECM und/oder zu einer Stimulierung der Aufbauprozesse der ECM führt, wodurch die Wirkung der erfindungsgemäßen Zusammensetzung bei der Bekämpfung der Hautalterung, zum Schutz der Haut vor Schäden durch ultraviolette Strahlung, zur Verbesserung der Elastizität der Haut und/oder zur Verringerung und/oder Verhinderung der Faltenbildung synergistisch verbessert wird. Erfindungsgemäß bevorzugt zu verwendende Polysaccharide sind solche, die mindestens einen Desoxyzucker-Baustein, insbesondere Fucose und/oder Rhamnose, enthalten, besonders bevorzugt ausgewählt aus Substanzen mit den INCl-Bezeichnungen Biosaccharide Gum-1, Biosaccharide Gum-2, Biosaccharide Gum-3 und Biosaccharide Gum-4, weiterhin Mischungen der vorgenannten, mindestens einen Desoxyzucker-Baustein enthaltenden Polysaccharide, beispielsweise der Mischung aus Biosaccharide Gum-2 und Biosaccharide Gum-3. Weitere erfindungsgemäß bevorzugt zu verwendende Polysaccharide sind ausgewählt aus den Glycosaminoglycanen, besonders bevorzugt Hyaluronsäure und deren Salze, Dextran, Dextransulfat, Chondroitin-4-sulfat und Chondroitin-6-sulfat. Weitere erfindungsgemäß bevorzugt zu verwendende Polysaccharide sind ausgewählt aus den Glucanen. Bei Glucanen oder Polyglucosanen handelt es sich um eine Klasse von Homopolysacchariden, deren Monomerbaustein ausschließlich Glucose ist. Beispiele für Glucane sind Cellulose, Amylose, Glycogen, Lichenin, Laminarin aus Algen, Pachyman aus Baumpilzen und Hefeglucane mit β-1,3-Bindung; Nigeran, ein aus Pilzen isoliertes Mycodextran (α-1,3-Glucan, α-1,4-Glucan), Pustulan (β-1,6-Glucan), Dextran, Curdlan (β-1,3-D-Glucan), Pullulan und Schizophyllan. Weitere erfindungsgemäß bevorzugt zu verwendende Polysaccharide, die in Gegenwart der erfindungsgemäßen Zusammensetzung, eine unerwartete Wirkungssteigerung zeigen, sind ausgewählt aus chemisch modifizierten Cellulose-Derivaten, vorzugsweise Hydroxyalkylcellulosen, wie Hydroxyethylcellulose, Hydroxypropylcellulose, Hydroxypropylmethylcellulose, Methylcellulose, Hydroxymethylcellulose, Carboxymethylcellulose, weiterhin insbesondere aus Stärken sowie Stärkeabbauprodukten wie Amylose und Amylopektin, chemisch und/oder thermisch modifizierte Stärken, z. B. Hydroxypropylstärkephosphat, Dihydroxypropyldistärkephosphat, Aluminiumstärkeoctenylsuccinat, Natriumstärkeoctenylsuccinat oder Calciumstärkeoctenylsuccinat, weiterhin Chitosan und dessen Salzen und anderen Derivaten, weiterhin aus Fucose- und Rhamnose-freien Polysacchariden, die Gums oder Gummen bilden, wie beispielsweise Guar-Gum, Xanthan-Gum, Alginate, insbesondere Natriumalginat, Gummi arabicum, Karaya-Gummi, Carrageenane, Johannisbrotkernmehl, Leinsamen-Gums, Schellack und Agar-Agar.

Erfindungsgemäß besonders bevorzugte Zusammensetzungen enthalten mindestens ein Polysaccharid in einer Gesamtmenge von 0,001 - 10 Gew.-%, bevorzugt 0,01 - 5 Gew.-%, besonders bevorzugt 0,1 - 3 Gew.-% und außerordentlich bevorzugt 0,5 - 1 Gew.-%, jeweils bezogen auf den Gehalt an Aktivsubstanz Polysaccharid in der gesamten Zusammensetzung, enthalten.

Erfindungsgemäß besonders bevorzugte Zusammensetzungen enthalten Hyaluronsäure(salz) und ein antioxidatives Peptid, ausgewählt aus (D-Arg)-Dmt-Lys-Phe-NH₂ und Dmt-(D-Arg)-Phe-Lys-NH₂. Weitere erfindungsgemäß besonders bevorzugte Zusammensetzungen enthalten ein Polysaccharid, ausgewählt aus beta-Glucan und/oder Natriumstärkeoctenylsuccinat, und ein Peptid, ausgewählt aus (D-Arg)-Dmt-Lys-Phe-NH₂ und Dmt-(D-Arg)-Phe-Lys-NH₂.

Weiterhin ist es erfindungsgemäß bevorzugt, die erfindungsgemäßen Zusammensetzungen mit Mischungen aus zwei oder mehreren der genannten Wirkstoffe, welche ausgewählt sind aus Aporphin-Alkaloiden, Purin und/oder Purinderivaten, natürlichen Betainverbindungen, alkyl- oder hydroxyalkylsubstituierten Harnstoffverbindungen, Monomeren, Oligomeren und Polymeren von Aminosäuren, N-C₂-C₂₄-Acylaminosäuren und/oder den Estern und/oder den physiologisch verträglichen Salzen dieser Substanzen, sowie Polysacchariden, einzusetzen.

Weitere erfindungsgemäß bevorzugte Zusammensetzungen enthalten mindestens einen weiteren Wirkstoff, ausgewählt aus Retinoiden, insbesondere Retinol und C₂-C₂₂-Fettsäureestern des Retinols, Oleanolsäure, Oleanol, Kreatin, Flavonoiden und Flavonoid-reichen Pflanzenextrakten, Isoflavonoiden und Isoflavonoid-reichen Pflanzenextrakten, Dihydroquercetin, Dillextrakt (Peucedanum Graveolens Extract), DNA- oder RNA-Oligonucleotiden, Vitaminen, Provitaminen und Vitaminvorstufen der Gruppen B und E und den Estern der vorgenannten Substanzen, α-Hydroxycarbonsäuren, α-Ketocarbonsäuren, β-Hydroxycarbonsäuren und deren Ester-, Lacton-und/oder Salzform, Silymarin, Ectoin, hautberuhigenden und sebumregulierenden Wirkstoffen sowie Mischungen der vorgenannten Substanzen.

Zu den Isoflavonoiden zählen erfindungsgemäß die Isoflavone und die Isoflavon-Glycoside. Unter Isoflavonen sind erfindungsgemäß Stoffe zu verstehen, die Hydrierungs-, Oxidations- oder Substitutionsprodukte des 3-Phenyl-4H-1-benzopyrans darstellen, wobei eine Hydrierung in der 2,3-Stellung des Kohlenstoffgerüsts vorliegen kann, eine Oxidation unter Ausbildung einer Carbonylgruppe in der 4-Stellung vorliegen kann, und unter Substitution der Ersatz eines oder mehrerer Wasserstoffatome durch Hydroxy- oder Methoxy-Gruppen zu verstehen ist. Zu den erfindungsgemäß bevorzugten Isoflavonen zählen beispielsweise Daidzein, Genistein, Prunetin, Biochanin, Orobol, Santal, Pratensein, Irigenin, Glycitein, Biochanin A und Formononetin. Besonders bevorzugte Isoflavone sind Daidzein, Genistein, Glycitein und Formononetin. Erfindungsgemäß besonders bevorzugte Isoflavon-Glycoside sind Daidzin und Genistin.

Zur Vitamin B-Gruppe oder zu dem Vitamin B-Komplex gehören unter anderem Vitamin B₁, Vitamin B₂, Vitamin B₃, insbesondere Nicotinsäure und Nicotinsäureamid (Niacinamid), Vitamin B₅, insbesondere Pantothensäure und Panthenol sowie Ester, Ether und kationische Derivate des Panthenols, außerdem Dihydro-3-hydroxy-4,4-dimethyl-2(3H)-furanon mit dem Trivialnamen Pantolacton; Vitamin B₆, wobei man hierunter keine einheitliche Substanz, sondern die unter den Trivialnamen Pyridoxin, Pyridoxamin und Pyridoxal bekannten Derivate des 5-Hydroxymethyl-2-methylpyridin-3-ols versteht, Vitamin B₇ (Biotin), Folsäure (Vitamin B₉, Vitamin B_{c}) und Orotsäure (Vitamin B₁₃). Zur Vitamin E-Gruppe zählen Tocopherol, insbesondere α-Tocopherol, und seine Derivate. Bevorzugte Derivate sind insbesondere die Ester, wie Tocopherylacetat, -nicotinat, -phosphat, -succinat, -linoleat, -oleat, Tocophereth-5, Tocophereth-10, Tocophereth-12, Tocophereth-18, Tocophereth-50 und Tocophersolan.

Erfindungsgemäß besonders bevorzugt zu verwendende kosmetische oder dermatologische Zusammensetzungen enthalten mindestens eine der folgenden Wirkstoffkombinationen:
Folsäure und ein Peptid, ausgewählt aus (D-Arg)-Dmt-Lys-Phe-NH₂ und Dmt-(D-Arg)-Phe-Lys-NH₂; Folsäure und N-Palmitoyl-Lys-Thr-Thr-Lys-Ser und ein Peptid, ausgewählt aus (D-Arg)-Dmt-Lys-Phe-NH₂ und Dmt-(D-Arg)-Phe-Lys-NH₂; Carnitin und Folsäure und ein Peptid, ausgewählt aus (D-Arg)-Dmt-Lys-Phe-NH₂ und Dmt-(D-Arg)-Phe-Lys-NH₂.

Erfindungsgemäß bevorzugte α-Hydroxycarbonsäuren oder α-Ketocarbonsäuren sind Glycolsäure, Milchsäure, Weinsäure, Citronensäure, 2-Hydroxybutansäure, 2,3-Dihydroxypropansäure, 2-Hydroxypentansäure, 2-Hydroxyhexansäure, 2-Hydroxyheptansäure, 2-Hydroxyoctansäure, 2-Hydroxydecansäure, 2-Hydroxydodecansäure, 2-Hydroxytetradecansäure, 2-Hydroxyhexadecansäure, 2-Hydroxyoctadecansäure, Mandelsäure, 4-Hydroxymandelsäure, Äpfelsäure, Erythrarsäure, Threarsäure, Glucarsäure, Galactarsäure, Mannarsäure, Gularsäure, 2-Hydroxy-2-methylbernsteinsäure, Gluconsäure, Brenztraubensäure, Glucuronsäure und Galacturonsäure. Besonders bevorzugte α-Hydroxycarbonsäuren sind Milchsäure, Citronensäure, Glycolsäure und Gluconsäure. Eine besonders bevorzugte β-Hydroxycarbonsäure ist Salicylsäure. Eine besonders bevorzugte α-Ketocarbonsäure ist Brenztraubensäure. Die Ester der genannten Säuren sind ausgewählt aus den Methyl-, Ethyl-, Propyl-, Isopropyl-, Butyl-, Amyl-, Pentyl-, Hexyl-, 2-Ethylhexyl-, Octyl-, Decyl-, Dodecyl- und Hexadecylestern. Weitere Derivate der vorgenannten Säuren sind deren physiologisch verträglichen Salze, bevorzugt die Zink-, Kupfer- und Mangansalze, die Salze der Alkali- und der Erdalkalimetalle und die Ammonium-, Alkylammonium-, Alkanolammonium- und Glucammoniumsalze, besonders bevorzugt die Natrium-, Kalium-, Magnesium- und Calcium-Salze. Die α-Hydroxycarbonsäuren, α-Ketocarbonsäuren oder β-Hydroxycarbonsäuren oder ihre Derivate sind bevorzugt in einer Gesamtmenge von 0,1 - 10 Gew.-%, besonders bevorzugt 0,5 - 5 Gew.-% und außerordentlich bevorzugt 1 - 2 Gew.-%, jeweils bezogen auf die gesamte Zusammensetzung, enthalten.

Weitere erfindungsgemäß besonders bevorzugte kosmetische oder dermatologische Zusammensetzungen enthalten Coenzym Q 10 und mindestens ein antioxidatives Peptid, ausgewählt aus (D-Arg)-Dmt-Lys-Phe-NH₂ und Dmt-(D-Arg)-Phe-Lys-NH₂. Dies hat den besonderen Vorteil, dass die auf die Mitochondrien gerichtete antioxidative Substanz in der Zusammensetzung nicht durch Luftsauerstoff bereits oxidiert und damit unbrauchbar wird.

Weitere erfindungsgemäß besonders bevorzugte kosmetische oder dermatologische Zusammensetzungen enthalten Ectoin und mindestens ein antioxidatives Peptid, ausgewählt aus (D-Arg)-Dmt-Lys-Phe-NH₂ und Dmt-(D-Arg)-Phe-Lys-NH₂.

Erfindungsgemäß bevorzugte hautberuhigende Wirkstoffe sind ausgewählt aus Allantoin, α-Bisabolol, α-Liponsäure, Extrakten aus Centella asiatica, Glycyrrethinsäure, dem Dipeptid Tyr-Arg, dessen Estern und dessen N-Acyl-Derivaten, insbesondere N-Acetyl-Tyr-Arg-hexyldecylester, sowie beliebigen Mischungen dieser Substanzen. Die hautberuhigenden Wirkstoffe sind bevorzugt in Mengen von 0,001 bis 5 Gew.-%, besonders bevorzugt 0,01 bis 2 Gew.-% und außerordentlich bevorzugt 0,1 bis 1 Gew.-%, jeweils bezogen auf die gesamte Zusammensetzung, enthalten.

Erfindungsgemäß bevorzugte sebumregulierende Wirkstoffe sind ausgewählt aus Azelainsäure, Sebacinsäure, 10-Hydroxydecansäure, 1,10-Decandiol, Potassium Azeloyl Diglycinate, Extrakten aus Spiraea Ulmaria, weiterhin aus wasser- und öllöslichen Extrakten aus Hamamelis, Klettenwurzel und Brennessel, Zimtbaumextract und Chrysanthemenextrakt. Die sebumregulierenden Wirkstoffe sind bevorzugt in einer Gesamtmenge von 0,001 bis 5 Gew.-%, besonders bevorzugt 0,01 bis 2 Gew.-% und außerordentlich bevorzugt 0,1 bis 1 Gew.-%, jeweils bezogen auf die gesamte Zusammensetzung, enthalten.

Erfindungsgemäß ist es bevorzugt, dass die Zusammensetzung eine topisch anzuwendende Zusammensetzung ist. So kann die Zusammensetzung auf geeignete Weise und ohne störende Nebenwirkungen auf andere Organe dort eingesetzt werden, wo sie ihre besonders geeignete Wirkung entfaltet, auf der Haut. Gemäß einer bevorzugten Ausführungsform der Erfindung ist die erfindungsgemäße Zusammensetzung, enthaltend ein antioxidatives Peptid gemäß Anspruch 1 sowie 0,001 bis 95 Gew.-% Wasser, bezogen auf das Gesamtgewicht der Zusammensetzung, in einer Form konfektioniert, die für eine Verabreichung auf topischem Wege geeignet ist.

In einer bevorzugten Ausführungsform, die für eine Verabreichung auf topischem Wege geeignet ist, enthalten die erfidungsgemäßem Zusammensetzungen mindestens einen konditionierenden Wirkstoff. Unter konditionierenden Wirkstoffen sind erfindungsgemäß solche Substanzen zu verstehen, die auf keratinische Materialien, insbesondere auf die Haut, aufziehen und die physikalischen und sensorischen Eigenschaften sowohl der Haut als auch des Produktes als solchem verbessern. Konditionierungsmittel glätten die oberste Schicht der Haut und machen sie weich und geschmeidig. Über die Auswahl der Konditionierungsmittel (fettig - weniger fettig, schnell oder langsam spreitend, schnell oder langsam in die Haut einziehend und so weiter) lässt sich das Hautgefühl des gesamten Produktes einstellen. Erfindungsgemäß bevorzugt zu verwendende konditionierende Wirkstoffe sind ausgewählt aus Fettstoffen, insbesondere pflanzlichen Ölen, wie Sonnenblumenöl, Olivenöl, Sojaöl, Rapsöl, Mandelöl, Jojobaöl, Orangenöl, Weizenkeimöl, Pfirsichkernöl und den flüssigen Anteilen des Kokosöls, Lanolin und seinen Derivaten, flüssigen Paraffinölen, Isoparaffin-ölen und synthetischen Kohlenwasserstoffen, Di-n-alkylethern mit insgesamt 12 bis 36 C-Atomen, z. B. Di-n-octylether und n-Hexyl-n-octylether, Fettsäuren, besonders linearen und/oder verzweigten, gesättigten und/oder ungesättigten C₈₋₃₀-Fettsäuren, Fettalkoholen, besonders gesättigten, ein- oder mehrfach ungesättigten, verzweigten oder unverzweigten Fettalkoholen mit 4 - 30 Kohlenstoffatomen, die mit 1 - 75, bevorzugt 5 - 20 Ethylenoxid-Einheiten ethoxyliert und/oder mit 3 - 30, bevorzugt 9 - 14 Propylenoxid-Einheiten propoxyliert sein können, Esterölen, das heißt Estern von C₆₋₃₀-Fettsäuren mit C₂₋₃₀-Fettalkoholen, Hydroxycarbonsäurealkylestern, Dicarbonsäureestern wie Di-n-butyladipat sowie Diolestern wie Ethylenglykoldioleat oder Propylenglykoldi(2-ethylhexanoat), symmetrischen, unsymmetrischen oder cyclischen Estern der Kohlensäure mit Fettalkoholen, z. B. Glycerincarbonat oder Dicaprylylcarbonat (Cetiol^{®} CC), Mono,- Di- und Trifettsäureestern von gesättigten und/oder ungesättigten linearen und/oder verzweigten Fettsäuren mit Glycerin, die mit 1 - 10, bevorzugt 7 - 9 Ethylenoxid-Einheiten ethoxyliert sein können, z. B. PEG-7 Glyceryl Cocoate, Wachsen, insbesondere Insektenwachsen, Pflanzenwachsen, Fruchtwachsen, Ozokerit, Mikrowachsen, Ceresin, Paraffinwachsen, Triglyceriden gesättigter und gegebenenfalls hydroxylierter C₁₆₋₃₀-Fettsäuren, z. B. gehärteten Triglyceridfetten, Phospholipiden, beispielsweise Sojalecithin, Ei-Lecithin und Kephalinen, Siliconverbindungen, ausgewählt aus Decamethylcyclopentasiloxan, Dodecamethylcyclohexasiloxan und Siliconpolymeren, die gewünschtenfalls quervernetzt sein können, z. B. Polydialkylsiloxanen, Polyalkylarylsiloxanen, ethoxylierten und/oder propoxylierten Polydialkylsiloxanen mit der früheren INCl-Bezeichnung Dimethicone Copolyol, sowie Polydialkylsiloxanen, die Amin- und/oder Hydroxy-Gruppen enthalten, bevorzugt Substanzen mit den INCl-Bezeichnungen Dimethiconol, Amodimethicone oder Trimethylsilylamodimethicone. Die Einsatzmenge der Fettstoffe beträgt bevorzugt 0,1 - 99 Gew.-%, besonders bevorzugt 2 - 50 Gew.-% und außerordentlich bevorzugt 5 - 20 Gew.-%, jeweils bezogen auf das gesamte Hautbehandlungsmittel.

In einer weiteren bevorzugten Ausführungsform kann die Wirkung der erfindungsgemäßen Zusammensetzung durch die Zugabe von einem oder mehreren Emulgatoren oder Tensiden gesteigert werden. Unter den Begriffen Tensid oder Emulgator werden oberflächenaktive Substanzen verstanden, die eine anionische oder kationische Ladung im Molekül tragen. Ebenfalls kann im Molekül sowohl eine anionische als auch kationische Ladung vorhanden sein. Diese zwitterionischen oder amphoteren oberflächenaktiven Substanzen können erfindungsgemäß ebenfalls eingesetzt werden. Weiterhin können die oberflächenaktiven Substanzen auch nicht-ionisch sein. Die erfindungsgemäßen Zusammensetzungen enthalten die Emulgatoren bevorzugt in Mengen von 0,1 - 25 Gew.-%, besonders bevorzugt 0,5 - 15 Gew.-%, außerordentlich bevorzugt 1 - 7 Gew.-%, jeweils bezogen auf die gesamte Zusammensetzung. Bevorzugte Emulgatoren und Tenside sind
- Anlagerungsprodukte von 4 bis 30 Mol Ethylenoxid und/oder 0 bis 5 Mol Propylenoxid an lineare Fettalkohole mit 8 bis 22 C-Atomen, an Fettsäuren mit 12 bis 22 C-Atomen und an Alkylphenole mit 8 bis 15 C-Atomen in der Alkylgruppe,
- C₁₂-C₂₂-Fettsäuremono- und -diester von Anlagerungsprodukten von 1 bis 30 Mol Ethylenoxid an Polyole mit 3 bis 6 Kohlenstoffatomen, insbesondere an Glycerin,
- Ethylenoxid- und Polyglycerin-Anlagerungsprodukte an Methylglucosid-Fettsäureester, Fettsäurealkanolamide und Fettsäureglucamide,
- C₈-C₂₂-Alkylmono- und -oligoglycoside und deren ethoxylierte Analoga, wobei Oligomerisierungsgrade von 1,1 bis 5, insbesondere 1,2 bis 2,0, und Glucose als Zuckerkomponente bevorzugt sind,
- Gemische aus Alkyl-(oligo)-glucosiden und Fettalkoholen zum Beispiel das im Handel erhältliche Produkt Montanov^{®}68,
- Anlagerungsprodukte von 5 bis 60 Mol Ethylenoxid an Rizinusöl und gehärtetes Rizinusöl,
- Partialester von Polyolen mit 3-6 Kohlenstoffatomen mit gesättigten Fettsäuren mit 8 bis 22 C-Atomen,
- Sterine. Als Sterine wird eine Gruppe von Steroiden verstanden, die am C-Atom 3 des Steroid-Gerüstes eine Hydroxylgruppe tragen und sowohl aus tierischem Gewebe (Zoosterine) wie auch aus pflanzlichen Fetten (Phytosterine) isoliert werden. Beispiele für Zoosterine sind das Cholesterin und das Lanosterin. Beispiele geeigneter Phytosterine sind Ergosterin, Stigmasterin und Sitosterin. Auch aus Pilzen und Hefen werden Sterine, die sogenannten Mykosterine, isoliert.
- Phospholipide. Hierunter werden vor allem die Glucose-Phospolipide, die z.B. als Lecithine bzw. Phosphatidylcholine aus z.B. Eidotter oder Pflanzensamen (z.B. Sojabohnen) gewonnen werden, verstanden.
- Fettsäureester von Zuckern und Zuckeralkoholen, wie Sorbit
- Polyglycerine und Polyglycerinderivate wie beispielsweise Polyglycerinpoly-12-hydroxystearat (Handelsprodukt Dehymuls^{®} PGPH)
anionische Tenside sind, jeweils in Form der Natrium-, Kalium- und Ammonium- sowie der Mono-, Di- und Trialkanolammoniumsalze mit 2 bis 4 C-Atomen in der Alkanolgruppe:
- lineare und verzweigte Fettsäuren mit 8 bis 30 C - Atomen und deren Na-, K-, Ammonium-, Ca-, Mg- und Zn - Salze.
- lineare und verzweigte Fettsäuren mit 8 bis 30 C-Atomen (Seifen),
- Ethercarbonsäuren der Formel R-O-(CH₂-CH₂O)ₓ-CH₂-COOH, in der R eine lineare Alkylgruppe mit 8 bis 30 C-Atomen und x = 0 oder 1 bis 16 ist,
- Acylsarcoside mit 8 bis 24 C-Atomen in der Acylgruppe,
- Acyltauride mit 8 bis 24 C-Atomen in der Acylgruppe,
- Acylisethionate mit 8 bis 24 C-Atomen in der Acylgruppe,
- Sulfobernsteinsäuremono- und -dialkylester mit 8 bis 24 C-Atomen in der Alkylgruppe und Sulfobernsteinsäuremono-alkylpolyoxyethylester mit 8 bis 24 C-Atomen in der Alkylgruppe und 1 bis 6 Oxyethylgruppen,
- lineare Alkansulfonate mit 8 bis 24 C-Atomen,
- lineare Alpha-Olefinsulfonate mit 8 bis 24 C-Atomen,
- Alpha-Sulfofettsäuremethylester von Fettsäuren mit 8 bis 30 C-Atomen,
- Alkylsulfate und Alkylpolyglykolethersulfate der Formel R-O(CH₂-CH₂O)ₓ-OSO₃H, in der R eine bevorzugt lineare Alkylgruppe mit 8 bis 30 C-Atomen und x = 0 oder 1 bis 12 ist,
- Gemische oberflächenaktiver Hydroxysulfonate gemäß DE-A-37 25 030,
- sulfatierte Hydroxyalkylpolyethylen- und/oder Hydroxyalkylenpropylenglykolether gemäß DE-A-37 23 354,
- Sulfonate ungesättigter Fettsäuren mit 8 bis 24 C-Atomen und 1 bis 6 Doppelbindungen gemäß DE-A-39 26 344,
- Ester der Weinsäure und Zitronensäure mit Alkoholen, die Anlagerungsprodukte von etwa 2-15 Molekülen Ethylenoxid und/oder Propylenoxid an Fettalkohole mit 8 bis 22 C-Atomen darstellen,
- Alkyl- und/oder Alkenyletherphosphate der Formel (VI), 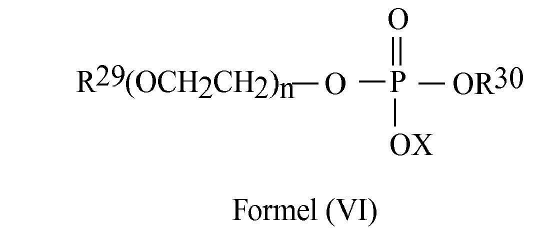 in der R²⁹ bevorzugt für einen aliphatischen Kohlenwasserstoffrest mit 8 bis 30 Kohlenstoffatomen, R³⁰ für Wasserstoff, einen Rest (CH₂CH₂O)ₙR²⁹ oder X, n für Zahlen von 1 bis 10 und X für Wasserstoff, ein Alkali- oder Erdalkalimetall oder NR³¹R³²R³³R³⁴, mit R³¹ bis R³⁴ unabhängig voneinander stehend für einen C₁ bis C₄ - Kohlenwasserstoffrest, steht,
- sulfatierte Fettsäurealkylenglykolester der Formel R³⁵CO(AlkO)ₙSO₃M, in der R³⁵CO- für einen linearen oder verzweigten, aliphatischen, gesättigten und/oder ungesättigten Acylrest mit 6 bis 22 C-Atomen, Alk für CH₂CH₂, CHCH₃CH₂ und/oder CH₂CHCH₃, n für Zahlen von 0,5 bis 5 und M für ein Kation steht, wie sie in der DE-OS 197 36 906.5 beschrieben sind,
- Monoglyceridsulfate und Monoglyceridethersulfate der Formel (VIII), 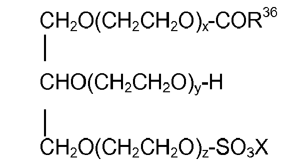 wie sie beispielsweise in EP 561825 B1, EP 561999 B1, DE 4204700 A1 oder von A.K.Biswas et al. in J.Am.Oil.Chem.Soc. 37, 171 (1960) und F.U.Ahmed in J.Am.Oil.Chem.Soc. 67, 8 (1990) beschrieben worden sind, in der R³⁶CO für einen linearen oder verzweigten Acylrest mit 6 bis 22 Kohlenstoffatomen, x, y und z in Summe für 0 oder für Zahlen von 1 bis 30, vorzugsweise 2 bis 10, und X für ein Alkali- oder Erdalkalimetall steht. Typische Beispiele für im Sinne der Erfindung geeignete Monoglycerid(ether)sulfate sind die Umsetzungsprodukte von Laurinsäuremonoglycerid, Kokosfettsäuremonoglycerid, Palmitinsäuremonoglycerid, Stearinsäuremonoglycerid, Ölsäuremonoglycerid und Talgfettsäuremonoglycerid sowie deren Ethylenoxidaddukte mit Schwefeltrioxid oder Chlorsulfonsäure in Form ihrer Natriumsalze. Vorzugsweise werden Monoglyceridsulfate der Formel (VIII) eingesetzt, in der R³⁶CO für einen linearen Acylrest mit 8 bis 18 Kohlenstoffatomen steht;
   N-Alkyl-N,N-dimethylammonium-glycinate, beispielsweise das Kokosalkyl-dimethylammoniumglycinat, N-Acyl-aminopropyl-N,N-dimethylammoniumglycinate, beispielsweise das Kokosacylaminopropyl-dimethylammoniumglycinat, und 2-Alkyl-3-carboxymethyl-3-hydroxyethyl-imidazoline mit jeweils 8 bis 18 C-Atomen in der Alkyl- oder Acylgruppe sowie das Kokosacylaminoethylhydroxyethylcarboxymethylglycinat;
   N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 24 C-Atomen in der Alkylgruppe, N-Kokosalkylaminopropionat, Kokosacylaminoethylaminopropionat, C₁₂ - C₁₈ - Acylsarcosin;
- Anlagerungsprodukten von 2 bis 30 Mol Ethylenoxid und/ oder 0 bis 5 Mol Propylenoxid an lineare Fettalkohole mit 8 bis 22 C-Atomen, an Fettsäuren mit 12 bis 22 C-Atomen und an Alkylphenole mit 8 bis 15 C-Atomen in der Alkylgruppe;
- C_{12/18}-Fettsäuremono- und -diestern von Anlagerungsprodukten von 1 bis 30 Mol Ethylenoxid an Glycerin;
- Glycerinmono- und -diestern und Sorbitanmono- und -diestern von gesättigten und ungesättigten Fettsäuren mit 6 bis 22 Kohlenstoffatomen und deren Ethylenoxidanlagerungsprodukten;
- Alkylmono- und -oligoglycosiden mit 8 bis 22 Kohlenstoffatomen im Alkylrest und deren ethoxylierten Analoga;
- Anlagerungsprodukten von 15 bis 60 Mol Ethylenoxid an Ricinusöl und/oder gehärtetes Ricinusöl;
- Polyolestern;
- Anlagerungsprodukten von 2 bis 15 Mol Ethylenoxid an Ricinusöl und/oder gehärtetes Ricinusöl;
- Partialestern auf Basis linearer, verzweigter, ungesättigter bzw. gesättigter C_{6/22}-Fettsäuren, Ricinolsäure sowie 12-Hydroxystearinsäure und Glycerin, Polyglycerin, Pentaerythrit, Dipentaerythrit, Zuckeralkoholen, Alkylglucosiden sowie Polyglucosiden;
- Trialkylphosphaten sowie Mono-, Di- und/oder Tri-PEG-Alkylphosphaten;
- Wollwachsalkoholen;
- Polysiloxan-Polyalkyl-Polyether-Copolymeren bzw. entsprechenden Derivaten;
- Mischestern aus Pentaerythrit, Fettsäuren, Citronensäure und Fettalkohol und/oder Mischestern von Fettsäuren mit 6 bis 22 Kohlenstoffatomen, Methylglucose und Polyolen sowie
- Polyalkylenglycolen;
- Anlagerungsprodukte von 2 bis 50 Mol Ethylenoxid und/oder 0 bis 5 Mol Propylenoxid an lineare und verzweigte Fettalkohole mit 8 bis 30 C-Atomen, an Fettsäuren mit 8 bis 30 C-Atomen und an Alkylphenole mit 8 bis 15 C-Atomen in der Alkylgruppe,
- mit einem Methyl- oder C₂ - C₆ - Alkylrest endgruppenverschlossene Anlagerungsprodukte von 2 bis 50 Mol Ethylenoxid und/oder 0 bis 5 Mol Propylenoxid an lineare und verzweigte Fettalkohole mit 8 bis 30 C-Atomen, an Fettsäuren mit 8 bis 30 C-Atomen und an Alkylphenole mit 8 bis 15 C-Atomen in der Alkylgruppe, wie beispielsweise die unter den Verkaufsbezeichnungen Dehydol^{®} LS, Dehydol^{®} LT (Cognis) erhältlichen Typen,
- C₁₂-C₃₀-Fettsäuremono- und -diester von Anlagerungsprodukten von 1 bis 30 Mol Ethylenoxid an Glycerin,
- Anlagerungsprodukte von 5 bis 60 Mol Ethylenoxid an Rizinusöl und gehärtetes Rizinusöl,
- Polyolfettsäureester, wie beispielsweise das Handelsprodukt Hydagen^{®} HSP (Cognis) oder Sovermol - Typen (Cognis),
- alkoxylierte Triglyceride,
- alkoxylierte Fettsäurealkylester der Formel R³⁷CO-(OCH₂CHR³⁸)_{w}OR³⁹, in der R³⁷CO für einen linearen oder verzweigten, gesättigten und/oder ungesättigten Acylrest mit 6 bis 22 Kohlenstoffatomen, R³⁸ für Wasserstoff oder Methyl, R³⁹ für lineare oder verzweigte Alkylreste mit 1 bis 4 Kohlenstoffatomen und w für Zahlen von 1 bis 20 steht,
- Aminoxide,
- Hydroxymischether, wie sie beipielsweise in der DE-OS 19738866 beschrieben sind,
- Sorbitanfettsäureester und Anlagerungeprodukte von Ethylenoxid an Sorbitanfettsäureester wie beispielsweise die Polysorbate,
- Zuckerfettsäureester und Anlagerungsprodukte von Ethylenoxid an Zuckerfettsäureester,
- Anlagerungsprodukte von Ethylenoxid an Fettsäurealkanolamide und Fettamine,
- Fettsäure-N-alkylglucamide,
- Alkylpolygykoside entsprechend der allgemeinen Formel RO-(Z)ₓ wobei R für Alkyl, Z für Zucker sowie x für die Anzahl der Zuckereinheiten steht. Die erfindungsgemäß verwendbaren Alkylpolyglykoside können lediglich einen bestimmten Alkylrest R enthalten. Üblicherweise werden diese Verbindungen aber ausgehend von natürlichen Fetten und Ölen oder Mineralölen hergestellt. In diesem Fall liegen als Alkylreste R Mischungen entsprechend den Ausgangsverbindungen bzw. entsprechend der jeweiligen Aufarbeitung dieser Verbindungen vor. Besonders bevorzugt sind solche Alkylpolyglykoside, bei denen R
- im wesentlichen aus C₈- und C₁₀-Alkylgruppen,
- im wesentlichen aus C₁₂- und C₁₄-Alkylgruppen,
- im wesentlichen aus C₈- bis C₁₆-Alkylgruppen oder
- im wesentlichen aus C₁₂- bis C₁₆-Alkylgruppen oder
- im wesentlichen aus C₁₆ bis C₁₈-Alkylgruppen besteht.

Als Zuckerbaustein Z können beliebige Mono- oder Oligosaccharide eingesetzt werden. Üblicherweise werden Zucker mit 5 bzw. 6 Kohlenstoffatomen sowie die entsprechenden Oligosaccharide eingesetzt. Solche Zucker sind beispielsweise Glucose, Fructose, Galactose, Arabinose, Ribose, Xylose, Lyxose, Allose, Altrose, Mannose, Gulose, Idose, Talose und Sucrose. Bevorzugte Zuckerbausteine sind Glucose, Fructose, Galactose, Arabinose und Sucrose; Glucose ist besonders bevorzugt. Die erfindungsgemäß verwendbaren Alkylpolyglykoside enthalten im Schnitt 1,1 bis 5 Zuckereinheiten. Alkylpolyglykoside mit x-Werten von 1,1 bis 2,0 sind bevorzugt. Ganz besonders bevorzugt sind Alkylglykoside, bei denen x 1,1 bis 1,8 beträgt. Auch die alkoxylierten Homologen der genannten Alkylpolyglykoside können erfindungsgemäß eingesetzt werden. Diese Homologen können durchschnittlich bis zu 10 Ethylenoxid- und/oder Propylenoxideinheiten pro Alkylglykosideinheit enthalten.

In einer besonders bevorzugten Ausführungsform ist mindestens ein nichtionischer Emulgator mit einem HLB-Wert von 8 und darunter enthalten. Derartige bevorzugte Emulgatoren sind insbesondere Verbindungen der allgemeinen Formel R¹ - O - R², in der R¹ eine primäre lineare Alkyl-, Alkenyl- oder Acylgruppe mit 20 - 30 C-Atomen, bevorzugt ein Behenyl-Rest, und R² Wasserstoff, eine Gruppe mit der Formel -(CₙH₂ₙO)ₓ-H mit x = 1 oder 2 und n = 2 - 4 oder eine Polyhydroxyalkylgruppe mit 4 - 6 C-Atomen und 2 - 5 Hydroxylgruppen, bevorzugt Wasserstoff, darstellt. Eine besonders bevorzugte Verbindung R¹ - O - R² ist Behenylalkohol. Weitere bevorzugt geeignete Emulgatoren mit einem HLB-Wert von 8 und darunter sind die Anlagerungsprodukte von 1 oder 2 Mol Ethylenoxid oder Propylenoxid an Behenylalkohol, Erucylalkohol, Arachidylalkohol oder auch an Behensäure oder Erucasäure. Bevorzugt eignen sich auch die Monoester von C₁₆-C₃₀-Fettsäuren mit Polyolen wie z. B. Pentaerythrit, Trimethylolpropan, Diglycerin, Sorbit, Glucose oder Methylglucose. Beispiele für solche Produkte sind z. B. Sorbitanmonobehenat oder Pentaerythritmonoerucat. Die Anwesenheit von Emulgatoren der allgemeinen Formel R¹ - O - R² kann zu lamellaren Strukturen in der Emulsion führen, die besonders günstige Effekte auf die Wiederherstellung der lamellaren Struktur der Haut haben können. Besonders bevorzugte erfindungsgemäße Zusammensetzungen sind daher **dadurch gekennzeichnet, dass** sie in Form einer lamellare Strukturen aufweisenden Öl-in-Wasser-Emulsion vorliegen.

Erfindungsgemäß bevorzugt sind ebenfalls kationische Tenside vom Typ der quartären Ammoniumverbindungen, der Esterquats und der Amidoamine. Bevorzugte quaternäre Ammoniumverbindungen sind Ammoniumhalogenide, insbesondere Chloride und Bromide, wie Alkyltrimethylammoniumchloride, Dialkyldimethylammoniumchloride und Trialkylmethylammoniumchloride, z. B. Cetyltrimethylammoniumchlorid, Stearyltrimethylammoniumchlorid, Distearyldimethylammoniumchlorid, Lauryldimethylammoniumchlorid, Lauryldimethylbenzylammoniumchlorid und Tricetylmethylammoniumchlorid, sowie die unter den INCl-Bezeichnungen Quaternium-27 und Quaternium-83 bekannten Imidazolium-Verbindungen. Die langen Alkylketten der oben genannten Tenside weisen bevorzugt 10 bis 18 Kohlenstoffatome auf.

Bei Esterquats handelt es sich um bekannte Stoffe, die sowohl mindestens eine Esterfunktion als auch mindestens eine quartäre Ammoniumgruppe als Strukturelement enthalten. Bevorzugte Esterquats sind quaternierte Estersalze von Fettsäuren mit Triethanolamin, quaternierte Estersalze von Fettsäuren mit Diethanolalkylaminen und quaternierten Estersalzen von Fettsäuren mit 1,2-Dihydroxypropyldialkylaminen. N,N-Bis(2-Palmitoyloxyethyl)dimethylammoniumchlorid ist ein Beispiel für solche Esterquats.

Die Alkylamidoamine werden üblicherweise durch Amidierung natürlicher oder synthetischer Fettsäuren und Fettsäureschnitte mit Dialkylaminoaminen hergestellt. Eine erfindungsgemäß besonders geeignete Verbindung aus dieser Substanzgruppe stellt das unter der Bezeichnung Tegoamid^{®} S 18 im Handel erhältliche Stearamidopropyl-dimethylamin dar.
Die kationischen Tenside sind in den erfindungsgemäßen Zusammensetzungen bevorzugt in Mengen von 0,05 bis 10 Gew.-%, bezogen auf das gesamte Mittel, enthalten. Mengen von 0,1 bis 5 Gew.-% sind besonders bevorzugt.

Bevorzugt können die erfindungsgemäßen Zusammensetzungen mindestens einen nichtionogenen Emulgator mit einem HLB-Wert von 8 bis 18, gemäß den im Römpp-Lexikon Chemie (Hrg. J. Falbe, M.Regitz), 10. Auflage, Georg Thieme Verlag Stuttgart, New York, (1997), Seite 1764, aufgeführten Definitionen enthalten. Nichtionogene Emulgatoren mit einem HLB-Wert von 10 - 15 können erfindungsgemäß besonders bevorzugt sein.

Unter den genannten Emulgatoren-Typen können die Emulgatoren, die kein Ethylenoxid und/oder Propylenoxid im Molekül enthalten ganz besonders bevorzugt sein.

Gemäß einer besonders bevorzugten Ausführungsform der Erfindung ist mindestens ein Tensid ausgewählt aus der Gruppe der nichtionischen Tenside. Es können neben dem mindestens einen nichtionischen Tensid auch weitere Tenside in der erfindungsgemäßen Zusammensetzung enthalten sein, die aus anderen, insbesondere den oben genannten Tensidklassen ausgewählt sind. Bevorzugte Zusammensetzungen, enthaltend mindestens ein nicht-ionisches Tensid, sind vorteilhafterweise besonders schonende und hautverträgliche kosmetische und/oder dermatologische Formulierungen, die eine geringe Hautreizung aufweisen, aber gleichzeitig die Penetration der antioxidativen Substanz in die Haut, insbesondere in die tieferen Hautschichten zu den Fibroblasten der Dermis, und dabei insbesondere in die Mitochondrien der Fibroblasten gewährleisten. Ihr Einsatz führt zu einer synergistischen Erhöhung der Wirksamkeit der erfindungsgemäßen antioxidativen Substanzen zur Bekämpfung der Hautalterung, zum Schutz der Haut vor Schäden durch ultraviolette Strahlung, zur Verbesserung der Elastizität der Haut und/oder zur Verringerung und/oder Verhinderung der Faltenbildung.

Bevorzugt liegen die erfindungsgemäßen kosmetischen oder dermatologischen Zusammensetzungen in Form einer flüssigen, fließfähigen oder festen Öl-in-Wasser-Emulsion, Wasser-in-Öl-Emulsion, Mehrfach-Emulsion, insbesondere einer Öl-in-Wasser-in-Öl- oder Wasser-in-Öl-in-Wasser-Emulsion, Makroemulsion, Miniemulsion, Mikroemulsion, PIT-Emulsion, Nanoemulsion, Pickering-Emulsion, Hydrodispersion, eines Hydrogels, eines Lipogels, einer ein- oder mehrphasigen Lösung, eines Schaumes, eines Puders oder einer Mischung mit mindestens einem als medizinischen Klebstoff geeigneten Polymer vor. Die Mittel können auch in nahezu wasserfreier Form, wie beispielsweise einem Öl oder einem Balsam, dargereicht werden. Hierbei kann der Träger ein pflanzliches oder tierisches Öl, ein Mineralöl, ein synthetisches Öl oder eine Mischung solcher Öle sein. In einer weiteren besonders bevorzugten Ausführungsform liegen die erfindungsgemäßen Zusammensetzungen in Form einer Wasser-in-Öl-Emulsion vor. Derartige Wasser-in-Öl-Emulsionen können so formuliert werden, dass die eine höhere Viskosität aufweisen, so dass sie sorgfältig in die Haut einzumassieren sind. Dies unterstützt die Wirkung der erfindungsgemäßen Zusammensetzungen. Genau so gut lassen sich aber auch niedrig-viskose Wasser-in-Öl-Emulsionen formulieren, insbesondere auf Basis des polymeren Wasser-in-Öl-Emulgators PEG-30 Dipolyhydroxystearat, erhältlich z. B. unter dem Handelsnamen Arlacel P 135 von Uniqema. Insbesondere mit Hilfe dieses Emulgators lassen sich sprühbare Wasser-in-Öl-Emulsionen formulieren.

Gemäß einer weiteren bevorzugten Ausführungsform der Erfindung ist in der erfindungsgemäßen Zusammensetzung weiterhin mindestens ein Hilfs- und/oder Zusatzstoff enthalten, ausgewählt aus Verdickungsmitteln und anorganischen oder organischen UV-Filtersubstanzen. Weitere bevorzugte Zusatzstoffe sind Antioxidantien, Konservierungsmittel, Lösungsmittel wie Ethanol, Isopropanol, Ethylenglykol, Propylenglykol, Propylenglykolmonoethylether, Glycerin und Diethylenglykol, Adsorbentien und Füllstoffe, wie Talkum und Veegum^{®}, Parfümöle, Pigmente sowie Farbstoffe zum Anfärben des Mittels, Substanzen zur Einstellung des pH-Wertes, Komplexbildner wie EDTA, NTA, β-Alanindiessigsäure und Phosphonsäuren, Treibmittel wie Propan-Butan-Gemische, Pentan, Isopentan, Isobutan, N₂O, Dimethylether, CO₂ und Luft.

Gemäß einer besonders bevorzugten Ausführungsform der Erfindung ist das mindestens eine antioxidative Peptid gemäß Anspruch 1 in Liposomen verkapselt. Dies hat den Vorteil, dass die Penetration durch die Epidemis in die Dermis und in die darin befindlichen Fibroblasten besonders gut erfolgen kann.

Ein weiterer Gegenstand der vorliegenden Erfindung ist die nicht-therapeutische, kosmetische Verwendung mindestens eines Peptids zur Bekämpfung der Hautalterung, zum Schutz der Haut vor Schäden durch ultraviolette und/oder infrarote Strahlung, zur Verbesserung der Elastizität der Haut und/oder zur Verringerung und/oder Verhinderung der Faltenbildung, **dadurch gekennzeichnet, dass** das Peptid ausgewählt ist aus Peptiden, welche
a) eine Länge von drei bis sechzehn Aminosäuren besitzen,
b) mindestens eine positive Nettoladung (bei pH 7,2) aufweisen und
c) mindestens zwei Aminosäuren mit aromatischen Seitenketten aufweisen, von denen mindestens eine Aminosäure ausgewählt ist aus Trp, Tyr, 2'-Methyltyrosin, 6'-Methyltyrosin, 2',6'-Dimethyltyrosin (Dmt), N,2',6'-Trimethyltyrosin (Tmt) oder 2'-Hydroxy-6'-Methyltyrosin (Hmt).

Ein weiterer Gegenstand der vorliegenden Erfindung ist die Verwendung mindestens eines Peptids zur Herstellung einer Zusammensetzung zur Bekämpfung der Hautalterung, zum Schutz der Haut vor Schäden durch ultraviolette und/oder infrarote Strahlung, zur Verbesserung der Elastizität der Haut und/oder zur Verringerung und/oder Verhinderung der Faltenbildung, **dadurch gekennzeichnet, dass** das Peptid ausgewählt ist aus Peptiden, welche
a) eine Länge von drei bis sechzehn Aminosäuren besitzen,
b) mindestens eine positive Nettoladung (bei pH 7,2) aufweisen und
c) mindestens zwei Aminosäuren mit aromatischen Seitenketten aufweisen, von denen mindestens eine Aminosäure ausgewählt ist aus Tyr, 2'-Methyltyrosin, 6'-Methyltyrosin, 2',6'-Dimethyltyrosin (Dmt), N,2',6'-Trimethyltyrosin (Tmt) oder 2'-Hydroxy-6'-Methyltyrosin (Hmt).

Für die erfindungsgemäßen Verwendungen gilt hinsichtlich bevorzugter Ausführungsformen mutatis mutandis das zu den erfindungsgemäßen Zusammensetzungen Gesagte.

Insbesondere werden die vorstehend beschriebenen erfindungsgemäßen Zusammensetzungen zur Bekämpfung der Hautalterung, zum Schutz der Haut vor Schäden durch ultraviolette und/oder infrarote Strahlung, zur Verbesserung der Elastizität der Haut und/oder zur Verringerung und/oder Verhinderung der Faltenbildung bzw. zur Herstellung einer Zusammensetzung zur Bekämpfung der Hautalterung, zum Schutz der Haut vor Schäden durch ultraviolette und/oder infrarote Strahlung, zur Verbesserung der Elastizität der Haut und/oder zur Verringerung und/oder Verhinderung der Faltenbildung verwendet.

Die nachfolgenden Formulierungsbeispiele sollen den Gegenstand der Erfindung erläutern, ohne ihn hierauf zu beschränken.

Alle Mengenangaben sind in Gew.-%, bezogen auf die gesamte Zusammensetzung.

### 1. Öl-in-Wasser-Emulsionen

1. Beispielserie:

| | 1 | 2 | 3 |
|---|---|---|---|
| Distelöl | 3,00 | 3,00 | 3,00 |
| Myritol 318 | 5,00 | 5,00 | 5,00 |
| Novata AB | 2,00 | 2,00 | 2,00 |
| Lanette 22 | 1,00 | 1,00 | 1,00 |
| Cutina MD | 2,00 | 2,00 | 2,00 |
| Stenol 1618 | 1,00 | 1,00 | 1,00 |
| Baysilon M350 | 1,00 | 1,00 | 1,00 |
| Controx KS | 0,05 | 0,05 | 0,05 |
| Propylparaben | 0,20 | 0,20 | 0,20 |
| Dry Flo Plus | 1,00 | 2,00 | 3,00 |
| Propylenglycol | 5,00 | 5,00 | 5,00 |
| Glycerine | 5,00 | 3,00 | 3,00 |
| Methylparaben | 0,20 | 0,20 | 0,20 |
| Phenoxyethanol | 0,9 | 0,5 | 0,9 |
| Herbasol Destillat Salbei | 1,0 | - | 1,0 |
| Herbasol Destillat Hamamelis | 1,0 | - | 1,0 |
| D-Panthenol | 0,7 | 0,525 | 0,6 |
| Hydroglycolic Extract of Ginseng | - | 0,45 | - |
| DSH-C N | 5,0 | 3,0 | - |
| (D-Arg)-Dmt-Lys-Phe-NH₂ | 0,01 | 0,10 | 0,1 |
| Parfum | 0,10 | 0,10 | 0,10 |
| Aqua | ad. 100 | ad. 100 | ad. 100 |

2. Beispielserie:

| | **1** | **2** | **3** | **4** |
|---|---|---|---|---|
| Lipoid S75-3 | 0,50 | 0,50 | 0,50 | 0,50 |
| Tocopherylacetat | 0,50 | 0,50 | 0,50 | 0,50 |
| Cutina MD | 1,00 | 1,00 | 1,00 | 1,00 |
| Lanette 22 | 2,00 | 2,00 | 2,00 | 2,00 |
| Baysilone M 350 | 0,50 | 0,50 | 0,50 | 0,50 |
| Propylparaben | 0,20 | 0,20 | 0,20 | 0,20 |
| Dow Corning 9040 | 1,00 | 1,00 | 1,00 | 1,00 |
| Glycerin | 4,50 | 3,00 | 3,00 | 3,00 |
| Hexandiol | 6,00 | 3,00 | - | 3,00 |
| Methylparaben | 0,20 | 0,20 | 0,20 | 0,20 |
| Tego Carbomer 140 | 0,30 | 0,30 | 0,30 | 0,30 |
| DS-HCN | 5,00 | 5,00 | 5,00 | 5,00 |
| Propylenglycol | 5,00 | 5,00 | 5,00 | 5,00 |
| Simulgel NS | 1,50 | 1,50 | 1,50 | 1,50 |
| Ti02 | 0,50 | 0,50 | 0,50 | 0,50 |
| Herbasol Destillat Salbei | - | 1,0 | - | - |
| Herbasol Destillat Hamamelis | - | 1,0 | - | - |
| D-Panthenol | - | 0,525 | 0,3 | 0,3 |
| Hydroglycolic Extract of Ginseng | - | - | 0,45 | 0,45 |
| (D-Arg)-Dmt-Lys-Phe-NH₂ | 0,001 | 0,1 | 0,01 | 0,01 |
| Wasser | Ad100 | Ad100 | Ad100 | Ad100 |

3. Beispielserie:

| | **1** | **2** | **3** |
|---|---|---|---|
| Sisterna SP30-C | 2,0 | 2,0 | 2,0 |
| Sisterna SP70-C | 0,8 | 0,8 | 0,8 |
| Propylparaben | 0,2 | 0,2 | 0,2 |
| Methylparaben | 0,2 | 0,2 | 0,2 |
| Ethanol | 5,0 | 3,0 | - |
| Dry Flo Plus | 1,0 | 1,0 | 1,0 |
| Parfum | 0,3 | 0,3 | 0,3 |
| Cetearyl Alcohol | 1,0 | 1,0 | 1,0 |
| Fucogel 1000 | 2,0 | 1,0 | 0,5 |
| Herbasol Destillat Salbei | 1,0 | 0,5 | 1,0 |
| Herbasol Destillat Hamamelis | 1,0 | 0,5 | - |
| D-Panthenol | 0,7 | - | - |
| Herbasol Destilat weisser Tee | - | 0,5 | 0,5 |
| Hydroglycolic Extract of Ginseng | - | 0,45 | 0,45 |
| Spirulina Extrakt 3002 | 1,00 | 1,00 | 1,00 |
| DSH-C N | 5,0 | 3,0 | - |
| Keltrol SF | 0,2 | - | 0,2 |
| Aristoflex AVC | - | 0,5 | - |
| Hexandiol-1,6 | 5,0 | - | - |
| Glycerin | 10,0 | 5,0 | 10,0 |
| Dow Corning 200 Fluid | 7,0 | - | - |
| Dow Corning 245 | - | 5,0 | 5,0 |
| Cetiol 868 | 2,0 | - | 2,0 |
| Dmt-(D-Arg)-Phe-Lys-NH₂ | 0,001 | 0,01 | 0,1 |
| Parfum | 0,3 | 0,3 | 0,3 |
| Wasser | Ad100 | Ad100 | Ad100 |

4. Beispielserie:

| | **1** | **2** | **3** |
|---|---|---|---|
| Dow Corning 245 Fluid | 25 | 25 | 25 |
| Abil EM 90 | 2 | 2 | 2 |
| Belsil DM 100 | 2,5 | 2,5 | 2,5 |
| Hydrogenated Castor Oil | 2 | 2,5 | 2 |
| Glycerin | 5 | 5 | 5 |
| NaCl | 2 | 2,1 | 2 |
| Symdiol 68 | 0,5 | 0,5 | 0,5 |
| Phenoxyethanol | 0,4 | 0,4 | 0,4 |
| D-Panthenol | 0,45 | 0,45 | 0,45 |
| D,I-alpha-bisabolol | 0,1 | 0,1 | 0,1 |
| Dmt-(D-Arg)-Phe-Lys-NH₂ | 0,001 | 0,01 | 0,1 |
| Parfum | 0,3 | 0,3 | 0,3 |
| Wasser | Ad100 | Ad100 | Ad100 |

5. Beispielserie:

| | **1** | **2** | **3** |
|---|---|---|---|
| Montanov 202 | 5,0 | 5,0 | 5,0 |
| Cutina MD | 2,0 | 2,0 | 2,0 |
| Cetearyl Alcohol | 1,0 | 1,0 | 1,0 |
| Cetiol B | 9,0 | 9,0 | 9,0 |
| Controx KS | 0,05 | 0,05 | 0,05 |
| Dow Corning 245 | - | 7,0 | 3,0 |
| Dow Corning 9040 | 1,0 | 1,0 | - |
| Parfum | 0,3 | 0,3 | 0,3 |
| Fucogel 1000 | - | 2,0 | - |
| Herbasol Destillat Salbei | - | - | 1,0 |
| Herbasol Destillat Hamamelis | - | - | 1,0 |
| D-Panthenol | - | 0,7 | 0,7 |
| Herbasol Destilat weisser Tee | - | 0,5 | - |
| Hydroglycolic Extract of Ginseng | - | 0,45 | - |
| DSH-C N | 5,0 | 5,0 | - |
| Keltrol SF | - | 0,2 | 0,2 |
| Aristoflex AVC | - | - | 0,5 |
| Hexandiol-1,6 | 6,0 | 6,0 | - |
| Glycerin | 3,0 | 5,0 | 10,0 |
| Dow Corning 200 Fluid | 7,0 | 7,0 | - |
| Dow Corning 245 | - | 5,0 | 3,0 |
| Dow Corning 9040 | 1,0 | - | 1,0 |
| Propylenglycol | 2,0 | 2,0 | - |
| Wasser | Ad100 | Ad100 | Ad100 |
| Tego Carbomer | 0,3 | 0,3 | 0,3 |
| Dry Flo Plus | 1,0 | 1,0 | 1,0 |
| Dmt-(D-Arg)-Phe-Lys-NH₂ | 0,001 | 0,01 | 0,1 |

6. Beispielserie:

| | **1** | **2** | **3** |
|---|---|---|---|
| Glucamate SSE-20 | 2,0 | 2,0 | 2,0 |
| Sympatens-BS/080 | 2,0 | 2,0 | 2,0 |
| Dry Flo Plus | - | 1,0 | 1,0 |
| Parfum | 0,3 | 0,3 | 0,3 |
| Silikonöl 350 | 0,3 | 0,3 | - |
| Cutina MD | 2,0 | 2,0 | 2,0 |
| Paraffinöl | 2,0 | 1,0 | - |
| Cetearyl Alcohol | 1,0 | 1,5 | 1,25 |
| Capryl-/Caprinsäure-Triglycerid | - | 3,5 | 3,0 |
| Herbasol Destillat Salbei | 1,0 | - | 1,0 |
| Herbasol Destillat Hamamelis | 1,0 | - | 1,0 |
| D-Panthenol | 0,7 | - | 1,0 |
| D,I-Bisabolol | 0,1 | 0,1 | - |
| Phenoxyethanol | 0,98 | 0,5 | 0,9 |
| Symdiol 68 | - | 0,5 | - |
| DSH-C N | - | 5,0 | - |
| Keltrol SF | - | - | 0,2 |
| Aristoflex AVC | - | 0,5 | 0,5 |
| Hexandiol-1,6 | - | 5,0 | 5,0 |
| Glycerin | 2,0 | 5,0 | 10,0 |
| Culminal MHPC 100 | 0,1 | 0,1 | 0,1 |
| Propylenglycol | 2,0 | 5,0 | 3,0 |
| Dmt-(D-Arg)-Phe-Lys-NH₂ | 0,001 | 0,01 | 0,1 |
| Wasser | Ad100 | Ad100 | Ad100 |

7. Hautcremes
Die Zusammensetzungen 1 - 4 sind bevorzugte Ausführungsformen von schützenden Tagescremes.

| | 1 | 2 | 3 | 4 |
|---|---|---|---|---|
| Distelöl | 3,00 | 3,00 | 3,00 | 3,00 |
| Myritol 318 | 5,00 | 5,00 | 5,00 | 5,00 |
| Novata AB | 2,00 | 2,00 | 2,00 | 2,00 |
| Behenyl Alcohol | 1,00 | 1,00 | 1,00 | 1,00 |
| Cutina MD | 2,00 | 2,00 | 2,00 | 2,00 |
| Cetearyl Alcohol | 1,00 | 1,00 | 1,00 | 1,00 |
| Isopropylstearat | 4,00 | 4,00 | 4,00 | 4,00 |
| Shea Butter | 2,00 | 2,00 | 2,00 | 2,00 |
| Baysilone-Öl M 350 | 1,00 | 1,00 | 1,00 | 1,00 |
| Controx KS | 0,05 | 0,05 | 0,05 | 0,05 |
| Propylparaben | 0,20 | 0,20 | 0,20 | 0,20 |
| Dow Corning 1501 Fluid | 1,00 | 1,00 | 1,00 | 1,00 |
| Dry Flo Plus | 1,00 | 1,00 | 1,00 | 1,00 |
| TiO₂ | 0,50 | 0,50 | 0,50 | 0,50 |
| Hexandiol | 6,00 | 3,00 | - | - |
| Propylenglycol | 5,00 | 5,00 | 5,00 | 5,00 |
| Glycerol | 5,00 | 3,00 | 3,00 | 3,00 |
| Methylparaben | 0,20 | 0,20 | 0,20 | 0,20 |
| Tego Carbomer | 0,40 | 0,40 | 0,40 | 0,40 |
| Dmt-(D-Arg)-Phe-Lys-NH₂ | 0,01 | 0,01 | 0,01 | 0,001 |
| Caomint | 1,00 | 0,50 | 2,00 | - |
| Calmosensine | 1,00 | 2,00 | - | 2,00 |
| Symdiol 68 | 0,30 | 0,50 | - | - |
| DSH-CN | 5,00 | 5,00 | 5,00 | 5,00 |
| Hydrovance | 4,30 | 2,50 | 8,60 | 10,00 |
| Kombuchka | 3,00 | - | - | 3,00 |
| Glistin | - | 2,00 | - | - |
| Natrulon RC 50 DG | - | - | 1,00 | - |
| Phytokine | 2,00 | 1,50 | 2,00 | 2,00 |
| Ridulisse C | 0,50 | 0,50 | 0,50 | 0,50 |
| Liftiline | 2,00 | 3,00 | 2,00 | 3,00 |
| Matrixyl 3000 | - | 2,00 | - | - |
| Perfume | 0,10 | 0,10 | 0,10 | 0,10 |
| Aqua | ad 100 | ad 100 | ad 100 | ad 100 |

8. Hautcremes:
Die Zusammensetzungen 1 - 3 sind weitere bevorzugte Ausführungsformen von schützenden Tagescremes für besonders anspruchsvolle Haut mit einem Gehalt an Antiageing-Wirkstoffen.

| | 1 | 2 | 3 |
|---|---|---|---|
| Cetearyl Isononanoate | 4,00 | 4,00 | 4,00 |
| Mineral oil | 6,00 | 6,00 | 6,00 |
| Cutina CBS | 2,00 | 2,00 | 2,00 |
| Palmitic Acid / Stearic Acid | 1,50 | 1,50 | 1,50 |
| Eumulgin B3 | 1,00 | 1,00 | 1,00 |
| Baysilone-Öl M 350 | 1,00 | 1,00 | 1,00 |
| Tocopheryl Acetate | 0,50 | 0,50 | 0,50 |
| Propylparaben | 0,30 | 0,30 | 0,30 |
| Pemulen TR 1 | 0,27 | 0,27 | 0,27 |
| Glycerin | 5,00 | 3,00 | 3,00 |
| Milchsäure 80 % | 0,26 | 0,26 | 0,26 |
| Propylenglycol | 5,00 | 5,00 | 5,00 |
| Methylparaben | 0,30 | 0,30 | 0,30 |
| Phenoxyethanol | 0,90 | 0,90 | 0,90 |
| Phytokine | 2,00 | 1,50 | 2,00 |
| Ridulisse C | 0,50 | 0,50 | 0,50 |
| Liftiline | 2,00 | 3,00 | 2,00 |
| Sepigel 305 | 0,50 | 0,50 | 0,50 |
| (D-Arg)-Dmt-Lys-Phe-NH₂ | 0,1 | 0,025 | 0,001 |
| Taurin | 1,00 | 0,50 | 2,00 |
| Calmosensine | 1,00 | 2,00 | - |
| Keltrol SF | 0,20 | 0,20 | 0,20 |
| N,N-Bis-(2-hydroxyethyl)harnstoff | 4,30 | 2,50 | 8,60 |
| Perfume | 0,30 | 0,30 | 0,30 |
| Natrium Ascorbylphosphat | 1,00 | - | 1,00 |
| Keratec Pep | - | 2,00 | - |
| Kreatin | - | - | 1,00 |
| Retinol | 0,10 | - | - |
| Deliner | - | 2,00 | - |
| Wasser | ad 100 | ad 100 | ad 100 |

9. Tagescremes:
Die Zusammensetzungen 1 - 5 sind bevorzugte Ausführungsformen von schützenden Tagescremes für besonders anspruchsvolle Haut mit einem Gehalt an Antiageing-Wirkstoffen.

| | 1 | 2 | 3 | 4 | 5 |
|---|---|---|---|---|---|
| Lipoid S 75-3 | 0,50 | 0,50 | 0,50 | 0,50 | 0,50 |
| Isopropylstearate | 4,00 | 4,00 | 4,00 | 4,00 | 4,00 |
| Dibutyl Adipate | 2,00 | 2,00 | 2,00 | 2,00 | 2,00 |
| Tocopheryl Acetate | 0,50 | 0,50 | 0,50 | 0,50 | 0,50 |
| Cutina MD | 1,00 | 1,00 | 1,00 | 1,00 | 1,00 |
| Behenyl Alcohol | 2,00 | 2,00 | 2,00 | 2,00 | 2,00 |
| Baysilone-Öl M 350 | 0,50 | 0,50 | 0,50 | 0,50 | 0,50 |
| Propylparaben | 0,20 | 0,20 | 0,20 | 0,20 | 0,20 |
| Dow Corning 9040 | 1,00 | 1,00 | 1,00 | 1,00 | 1,00 |
| Glycerol | 3,00 | 4,50 | 3,00 | 3,00 | 3,00 |
| Hexandiol | 6,00 | 3,00 | - | - | 4,00 |
| Methylparaben | 0,20 | 0,20 | 0,20 | 0,20 | 0,20 |
| Tego Carbomer | 0,30 | 0,30 | 0,30 | 0,30 | 0,30 |
| Algae Extract | 1,00 | - | - | - | - |
| Photosomes | 0,20 | 0,20 | 0,20 | 0,20 | 0,20 |
| Dimethylmethoxy Chromanol | 0,01 | 0,01 | 0,01 | 0,01 | 0,01 |
| Propylenglycol | 5,00 | 5,00 | 5,00 | 5,00 | 5,00 |
| Phytokine | 2,00 | 1,50 | 2,00 | 2,00 | 1,50 |
| Ridulisse C | 0,50 | 0,50 | 0,50 | 0,50 | 0,50 |
| Liftiline | 2,00 | 3,00 | 2,00 | 2,00 | 3,00 |
| Dmt-(D-Arg)-Phe-Lys-NH2 | 0,01 | 0,10 | 0,050 | 0,01 | 0,0020 |
| Calmosensine | 1,00 | 2,00 | - | 1,00 | 1,00 |
| Kombuchka | 3,00 | 3,00 | - | - | 2,00 |
| Natrium Ascorbylphosphat | - | - | 1,00 | 1,00 | 1,00 |
| Natrulon RC50DG | - | - | - | - | 1,00 |
| Deliner | - | - | 2,00 | 1,00 | - |
| Matrixyl 3000 | 1,00 | 1,00 | - | - | 1,00 |
| Simulgel NS | 1,50 | 1,50 | 1,50 | 1,50 | 1,50 |
| Sodium Benzoate | - | 0,20 | 0,50 | - | - |
| Phenoxyethanol | 0,50 | - | - | - | - |
| Symdiol 68 | 0,30 | 0,50 | 0,30 | - | - |
| TiO₂ | 0,50 | 0,50 | - | - | - |
| Hydrovance | 2,50 | 4,30 | 8,60 | 8,60 | 8,60 |
| Silymarin Phytosome | 0,30 | - | - | - | 0,50 |
| Ectoin | 0,50 | - | 0,50 | 0,50 | 0,50 |
| Vitamin B6 | - | - | 0,01 | 0,01 | 0,01 |
| Perfume | 0,35 | 0,35 | 0,35 | 0,35 | 0,35 |
| Aqua | ad 100 | ad 100 | ad 100 | ad 100 | ad 100 |

10. Pflegecremes
Die Zusammensetzungen 1 - 6 sind bevorzugte Ausführungsformen von schützenden Pflegecremes mit gel-ähnlicher Konsistenz.

| | 1 | 2 | 3 | 4 | 5 | 6 |
|---|---|---|---|---|---|---|
| 2-Ethylhexylpalmitat | 2,00 | 2,00 | 2,00 | 2,00 | 2,00 | 2,00 |
| Dow Corning 1403 Fluid | 5,00 | 5,00 | 5,00 | 5,00 | 5,00 | 5,00 |
| Synperonic PE L 64 | 2,00 | 2,00 | 2,00 | 2,00 | 2,00 | 2,00 |
| Dow Corning 9040 | 5,00 | 5,00 | 5,00 | 5,00 | 5,00 | 5,00 |
| Dipropylenglykol | 5,00 | 5,00 | 5,00 | 5,00 | 5,00 | 5,00 |
| Tego Carbomer 140 | 0,70 | 0,70 | 0,70 | 0,70 | 0,70 | 0,70 |
| Parfum | 0,35 | 0,30 | 0,35 | 0,35 | 0,30 | 0,30 |
| Phenoxyethanol, rein | 0,40 | 0,40 | 0,40 | 0,40 | 0,40 | 0,40 |
| Natrulon RC-50DG | - | 0,75 | 0,75 | 0,75 | 0,75 | 0,75 |
| Menthyl Lactate | 0,50 | 0,50 | 0,50 | 0,50 | 0,50 | 0,50 |
| Ethanol 96 % DEP vergällt | 5,00 | 5,00 | 5,00 | 5,00 | 5,00 | 5,00 |
| Coffein wasserfrei | 0,50 | - | 0,50 | 0,50 | 0,50 | 0,50 |
| Ridulisse C | - | - | - | 0,50 | - | - |
| Dmt-(D-Arg)-Phe-Lys-NH₂ | 0,0050 | 0,0050 | 0,0050 | 0,01 | 0,01 | 0,01 |
| Simulgel NS | 0,75 | 0,75 | 0,75 | 0,75 | 0,75 | 0,75 |
| Matrixyl | - | - | - | - | 2,00 | - |
| Matrixyl 3000 | - | - | - | - | - | 2,00 |
| Natriumhydroxid Perlen | 0,081 | 0,081 | 0,081 | 0,081 | 0,081 | 0,081 |
| Wasser, vollentsalzt | ad 100,00 | ad 100,00 | ad 100,00 | ad 100,00 | ad 100,00 | ad 100,00 |

11. Die Zusammensetzungen 1 - 5 sind bevorzugte Ausführungsformen von schützenden Tagescremes mit höherem Lichtschutzfilter (SPF ca. 10 - 15) für besonders anspruchsvolle Haut mit einem Gehalt an Antiageing-Wirkstoffen.

| | 1 | 2 | 3 | 4 | 5 |
|---|---|---|---|---|---|
| Montanov 68 | 5,00 | 5,00 | 5,00 | 5,00 | 5,00 |
| Myritol 318 | 5,00 | 5,00 | 5,00 | 5,00 | 5,00 |
| Cetiol SB 45 | 0,50 | 0,50 | 0,50 | 0,50 | 0,50 |
| Novata AB | 2,00 | 2,00 | 2,00 | 2,00 | 2,00 |
| Stenol 1618 | 1,00 | 1,00 | 1,00 | 1,00 | 1,00 |
| Baysilon M 350 | 0,50 | 0,50 | 0,50 | 0,50 | 0,50 |
| Tocopherylacetat | 0,50 | 0,50 | 0,50 | 0,50 | 0,50 |
| Controx KS | 0,25 | 0,25 | 0,25 | 0,25 | 0,25 |
| Parsol SLX | - | 4,00 | 4,00 | - | - |
| Parsol HS | - | 2,00 | 2,00 | 2,00 | - |
| Neo Heliopan AP | - | - | - | 1,00 | - |
| Parsol 340 | - | - | - | 5,00 | - |
| Uvinul MBC 95 | 2,00 | - | - | - | - |
| Parsol 1789 | 1,00 | 1,80 | 1,80 | - | - |
| Propylparaben | 0,20 | 0,20 | 0,20 | 0,20 | 0,20 |
| Tego Carbomer 140 | 0,50 | 0,50 | 0,50 | 0,50 | 0,50 |
| 1,6-Hexandiol | 6,00 | 6,00 | 3,00 | 6,00 | - |
| Talkum | 0,50 | 0,50 | 0,50 | 0,50 | 0,50 |
| Methylparaben | 0,20 | 0,20 | 0,20 | 0,20 | 0,20 |
| Glycerin | 4,50 | 4,50 | 3,00 | 4,50 | 3,00 |
| Dry Flo Plus | 1,00 | 1,00 | 1,00 | 1,00 | 1,00 |
| Natipide 2 PG | 1,00 | 1,00 | 1,00 | 1,00 | 1,00 |
| Phenoxyethanol | 0,40 | 0,40 | 0,40 | 0,40 | 0,40 |
| (D-Arg)-Dmt-Lys-Phe-NH₂ | 0,01 | 0,0050 | 0,01 | 0,01 | 0,01 |
| Carnitin | - | 0,10 | 0,50 | - | - |
| Matrixyl 3000 | 1,00 | - | - | 4,00 | 2,00 |
| Ridulisse C | - | - | 3,00 | - | 0,50 |
| Parfum | 0,40 | 0,40 | 0,40 | 0,40 | 0,40 |
| Aqua | ad 100 | ad 100 | ad 100 | ad 100 | ad 100 |

12. Wasser-in-Öl-Emulsion
Die Zusammensetzungen 1 - 3 sind bevorzugte Ausführungsformen von schützenden Cremes auf der Basis einer reichhaltigen Wasser-in-Öl-Emulsion, die in die Haut einmassiert werden kann und dadurch einen zusätzlichen Bonuseffekt hervorruft.

| | 1 | 2 | 3 |
|---|---|---|---|
| Lameform TGI | 3,00 | 3,00 | 3,00 |
| Elfacos ST 37 | 0,50 | 0,50 | 0,50 |
| Microcrystalline Wax | 3,00 | 3,00 | 3,00 |
| Softisan 649 | 1,00 | 1,00 | 1,00 |
| Paraffinöl | 8,00 | 8,00 | 8,00 |
| Vaseline | 2,00 | 2,00 | 2,00 |
| Vitamin E Acetat | 2,00 | 2,00 | 2,00 |
| Methylparaben | 0,30 | 0,30 | 0,30 |
| Propylparaben | 0,30 | 0,30 | 0,30 |
| Isopropylisostearat | 8,00 | 8,00 | 8,00 |
| 1,3-Butylenglycol | 5,00 | 6,00 | 7,00 |
| Dmt-(D-Arg)-Phe-Lys-NH₂ | 0,0050 | 0,01 | 0,01 |
| Carnitin | - | 0,10 | 0,50 |
| Milchsäure 80%ig | 0,60 | 0,60 | 0,60 |
| Panthenol | 0,5 | 1,0 | 0,5 |
| Tephrosia pupurea Seed Extract | 0,1 | - | - |
| Hydrovance | 2,00 | 4,00 | - |
| Parfum | 0,2 | 0,2 | 0,2 |
| Water | ad 100 | ad 100 | ad 100 |

13. Die Zusammensetzungen 1 - 3 sind bevorzugte Ausführungsformen von schützenden Gesichtswässern, die bevorzugt mit einem Pad, Bausch oder Tuch auf die Haut appliziert werden.

| | 1 | 2 | 3 |
|---|---|---|---|
| Dipropylenglycol | 10,00 | 10,00 | 10,00 |
| Chlorhexidindigluconat | 1,00 | 1,00 | 1,00 |
| Synperonic PE7 L 64 | 3,00 | 3,00 | 3,00 |
| D-Panthenol | 0,50 | 0,50 | 0,50 |
| Hydagen CMF | 3,00 | 3,00 | 3,00 |
| PEG-40 Hydrogenated Castor Oil / Trideceth 9 / Propylene Glycol | 0,50 | 0,50 | 0,50 |
| Caomint | 0,50 | 0,50 | 0,50 |
| (D-Arg)-Dmt-Lys-Phe-NH₂ | 0,01 | 0,0025 | 0,0050 |
| Ridulisse C | 1,00 | - | - |
| Fucogel 1000 | - | - | 1,00 |
| Betain | - | 1,00 | - |
| Parfum | 0,20 | 0,20 | 0,20 |
| Aqua | ad 100 | ad 100 | ad 100 |

14. Die Zusammensetzungen 1 - 3 sind bevorzugte Ausführungsformen von schützenden Cremes auf der Basis einer reichhaltigen und dennoch nicht-fettenden Wasser-in-Silicon-Emulsion, die in die Haut einmassiert werden kann.

| | 1 | 2 | 3 |
|---|---|---|---|
| Belsil DM 100 | 2,50 | 2,50 | 2,50 |
| Dow Corning 245 Fluid | 25,00 | 25,00 | 25,00 |
| Abil EM 90 | 2,00 | 2,00 | 2,00 |
| Natriumchlorid | 2,00 | 2,00 | 2,00 |
| Symdiol 68 | 0,30 | 0,30 | 0,30 |
| Phenoxyethanol | 0,40 | 0,40 | 0,40 |
| (D-Arg)-Dmt-Lys-Phe-NH₂ | 0,01 | 0,01 | 0,01 |
| Ridulisse C | - | 2,00 | 1,00 |
| Rhamnosoft | 2,00 | - | - |
| Hydrovance | - | 3,00 | - |
| Folsäure | - | - | 0,10 |
| Parfum | 0,30 | 0,30 | 0,30 |
| Aqua | ad 100 | ad 100 | ad 100 |

15. Beispielserie:
Die Zusammensetzungen 1 - 4 sind bevorzugte Ausführungsformen von schützenden Tagescremes für besonders anspruchsvolle Haut mit einem erhöhten Gehalt an diversen Antiageing-Wirkstoffen.

| | 1 | 2 | 3 | 4 |
|---|---|---|---|---|
| Distelöl | 3,00 | 3,00 | 3,00 | 3,00 |
| Myritol 318 | 5,00 | 5,00 | 5,00 | 5,00 |
| Novata AB | 2,00 | 2,00 | 2,00 | 2,00 |
| Behenyl Alcohol | 1,00 | 1,00 | 1,00 | 1,00 |
| Cutina MD | 2,00 | 2,00 | 2,00 | 2,00 |
| Cetearyl Alcohol | 1,00 | 1,00 | 1,00 | 1,00 |
| Isopropylstearat | 4,00 | 4,00 | 4,00 | 4,00 |
| Shea Butter | 2,00 | 2,00 | 2,00 | 2,00 |
| Baysilone-Öl M 350 | 1,00 | 1,00 | 1,00 | 1,00 |
| Controx KS | 0,05 | 0,05 | 0,05 | 0,05 |
| Propylparaben | 0,20 | 0,20 | 0,20 | 0,20 |
| Dow Corning 1501 Fluid | 1,00 | 1,00 | 1,00 | 1,00 |
| Dry Flo Plus | 1,00 | 1,00 | 1,00 | 1,00 |
| TiO₂ | 0,50 | 0,50 | 0,50 | 0,50 |
| Hexandiol | 6,00 | 3,00 | - | - |
| Propylenglycol | 5,00 | 5,00 | 5,00 | 5,00 |
| Glycerol | 5,00 | 3,00 | 3,00 | 3,00 |
| Methylparaben | 0,20 | 0,20 | 0,20 | 0,20 |
| Tego Carbomer | 0,40 | 0,40 | 0,40 | 0,40 |
| Algenextrakt | 1,00 | - | - | - |
| Symdiol 68 | 0,30 | 0,50 | - | - |
| DSH-CN | 5,00 | 5,00 | 5,00 | 5,00 |
| Hydrovance | 4,30 | 2,50 | 8,60 | 10,00 |
| Dmt-(D-Arg)-Phe-Lys-NH₂ | 0,01 | 0,02 | 0,015 | 0,02 |
| Natrulon RC 50 DG | - | - | 1,00 | - |
| Phytokine | 2,00 | - | - | - |
| Matrixyl 3000 | - | 2,00 | - | - |
| Ridulisse C | - | - | 2,00 | 2,50 |
| Argireline | 1,00 | - | - | - |
| Sepilift DPHP | - | 0,10 | - | - |
| Ederline H | 2,00 | - | - | - |
| Perfume | 0,10 | 0,10 | 0,10 | 0,10 |
| Aqua | ad 100 | ad 100 | ad 100 | ad 100 |

16. Tagescreme:
Die folgende Zusammensetzung ist eine weitere bevorzugte Ausführungsform einer schützenden erfindungsgemäßen Zusammensetzung.

| | |
|---|---|
| Lipoid S 75-3 | 1,000000 |
| Isopropylstearat | 2,000000 |
| Cetiol B | 4,000000 |
| Vitamin E Acetat | 0,500000 |
| Cutina MD | 1,000000 |
| Cetearyl Alcohol | 2,500000 |
| NOVATA AB PH | 0,500000 |
| Behenylalkohol | 3,500000 |
| Silikonöl 350 cs | 1,500000 |
| Propylparaben | 0,200000 |
| Dow Corning 9040 | 2,000000 |
| Glycerin 86% | 8,000000 |
| Hexandiol-1,6 | 6,000000 |
| Methylparaben | 0,200000 |
| Talkum | 3,000000 |
| Tego Carbomer 140 | 0,400000 |
| Hydroglycolic Extract of Caviar | 1,000000 |
| Dmt-(D-Arg)-Phe-Lys-NH2 | 0,00500000 |
| Ederline L | 1,000000 |
| DSH-C N | 3,000000 |
| Photosomes | 0,200000 |
| Lipochroman-6 | 0,010000 |
| Propandiol-1,2 | 4,000000 |
| Matrixyl 3000 | 3,000000 |
| Phenoxyethanol, rein | 0,400000 |
| Parfum | 0,200000 |
| RONASPHERELDP | 1,000000 |
| FD&C Yellow No. 6 | 0,000700 |
| Simulgel NS | 1,500000 |
| Natriumhydroxid | 0,040000 |
| Wasser, vollentsalzt | ad 100,00000 |

17. Nachtcreme:
Die folgende Zusammensetzung ist eine weitere bevorzugte Ausführungsform einer schützenden erfindungsgemäßen Zusammensetzung.

| | |
|---|---|
| Montanov 68 | 3,000000 |
| Capryl-/Caprinsäure-Triglycerid | 2,000000 |
| Cetiol SN | 8,000000 |
| Safloröl raffiniert | 3,500000 |
| Cetearyl Alcohol | 2,500000 |
| Cutina MD | 1,700000 |
| Behenylalkohol | 3,000000 |
| Cetiol SB 45 | 4,000000 |
| NOVATA AB PH | 0,500000 |
| Silikonöl 350 cs | 2,500000 |
| Controx KS | 0,050000 |
| Propylparaben | 0,200000 |
| Glycerin 86% | 9,000000 |
| Hexandiol-1,6 | 6,000000 |
| Methylparaben | 0,200000 |
| Tego Carbomer 140 | 0,300000 |
| Dmt-(D-Arg)-Phe-Lys-NH₂ | 0,020000 |
| Phenoxyethanol, rein | 0,400000 |
| Ultrasomes | 0,100000 |
| Matrixyl 3000 | 3,000000 |
| Parfum | 0,100000 |
| FD&C Yellow No. 6 | 0,000700 |
| Aluminiumstärkeoctenylsuccinat | 1,800000 |
| Simulgel EPG | 0,400000 |
| Natriumhydroxid | 0,031000 |
| Wasser, vollentsalzt | ad 100,000000 |

18. Hautcremes auf Basis einer Lipoprotein-Creme
Die Zusammensetzungen 1 - 6 sind bevorzugte Ausführungsformen von schützenden Cremes mit besonderer Hautverträglichkeit und einem Gehalt an diversen Antiageing-Wirkstoffen, auf Basis einer besonders milden Emulsionsbasis mit einem natürlichen Protein-Emulgator (Hibiscin^{®} HP LS 9198).

| | 1 | 2 | 3 | 4 | 5 | 6 |
|---|---|---|---|---|---|---|
| Myritol^{®} PC | 3,5 | 3,5 | 3,5 | 3,5 | 3,5 | 3,5 |
| Lanette^{®} 22 | 3,0 | 3,0 | 3,0 | 3,0 | 3,0 | 3,0 |
| Cutina^{®} GMS-V | 3,0 | 3,0 | 3,0 | 3,0 | 3,0 | 3,0 |
| Stenol^{®} 16/18 | 2,0 | 2,0 | 2,0 | 2,0 | 2,0 | 2,0 |
| Distelöl | 3,0 | 3,0 | 3,0 | 3,0 | 3,0 | 3,0 |
| Controx^{®} KS | 0,05 | 0,05 | 0,05 | 0,05 | 0,05 | 0,05 |
| Baysilon^{®} M350 | 1,0 | 1,0 | 1,0 | 1,0 | 1,0 | 1,0 |
| Isopropylstearat | 6,0 | 6,0 | 6,0 | 6,0 | 6,0 | 6,0 |
| Propylparaben | 0,2 | 0,2 | 0,2 | 0,2 | 0,2 | 0,2 |
| 1,3-Butylenglycol | 5,0 | 5,0 | 5,0 | 5,0 | 5,0 | 5,0 |
| 1,6-Hexandiol | 5,0 | 5,0 | 5,0 | 5,0 | 5,0 | 5,0 |
| Methylparaben | 0,2 | 0,2 | 0,2 | 0,2 | 0,2 | 0,2 |
| Hibiscin^{®} HP LS 9198 | 3,0 | 3,0 | 3,0 | 3,0 | 3,0 | 3,0 |
| Citronensäure | 0,1 | 0,1 | 0,1 | 0,1 | 0,1 | 0,1 |
| Simulgel^{®} NS | 2,0 | 2,0 | 2,0 | 2,0 | 2,0 | 2,0 |
| Pearl Protein Extract | - | - | 2,00 | - | - | 1,00 |
| (D-Arg)-Dmt-Lys-Phe-NH₂ | 0,02 | 0,01 | 0,01 | 0,01 | 0,01 | 0,01 |
| Ridulisse C | 1,00 | 0,50 | 1,00 | - | - | - |
| Carnitin | 0,20 | - | - | 0,50 | 0,30 | 0,30 |
| Matrixyl 3000 | 3,00 | - | - | - | 2,00 | - |
| Aqua | ad 100 | ad 100 | ad 100 | ad 100 | ad 100 | ad 100 |

19. Reinigungszubereitungen:

| | **1** | **2** | **3** |
|---|---|---|---|
| Dipropylenglycol | 10,00 | 10,00 | 10,00 |
| Chlorhexidindigluconat | 1,00 | 1,00 | 1,00 |
| Synperonic PE7L 64 | 3,00 | 3,00 | 3,00 |
| D-Panthenol | 0,50 | 0,50 | 0,50 |
| Hydagen CMF | 3,00 | 3,00 | 3,00 |
| PEG-40 Hydrogenated Castor Oil / Trideceth 9 / Propylen Glycol | 0,50 | 0,50 | 0,50 |
| (D-Arg)-Dmt-Lys-Phe-NH₂ | 0,005 | 0,01 | 0,1 |
| Parfume | 0,20 | 0,20 | 0,20 |
| Aqua | ad. 100 | ad. 100 | ad. 100 |

20. Reinigungsgele mit schützendem Effekt:
Die Zusammensetzungen 1 - 3 sind bevorzugte Ausführungsformen von Reinigungsgelen mit einem schützenden Effekt zur Komplettierung der Hautpflege.

| | 1 | 2 | 3 |
|---|---|---|---|
| Carbomer | 1,40 | 1,40 | 1,40 |
| 1,3-Butylenglycol | 4,00 | 4,00 | 4,00 |
| Sodium Benzoate | 0,40 | 0,40 | 0,40 |
| Plantaren^{®} 1200 | 7,50 | 7,50 | 7,50 |
| Dehyton^{®} K | 3,40 | 3,40 | 3,40 |
| Texapon^{®} SB 3 | 5,00 | 5,00 | 5,00 |
| Cetiol^{®} HE | 0,50 | 0,50 | 0,50 |
| Lamesoft^{®} PO 65 | 5,00 | 5,00 | 5,00 |
| Controx KS | 0,05 | 0,05 | 0,05 |
| Sodium pyrrolidone carboxylic acid | 1,60 | 1,60 | - |
| Pantolactone | 1,00 | 1,00 | - |
| Tetrasodium EDTA | 0,25 | 0,25 | 0,25 |
| Sodium Lactate | 1,80 | | |
| Panthenol | 0,50 | 0,50 | 0,50 |
| Dmt-(D-Arg)-Phe-Lys-NH₂ | 0,01 | 0,01 | 0,01 |
| Ridulisse C | 1,00 | - | - |
| Carnitin | 0,20 | - | 0,20 |
| Perfume | 0,40 | 0,40 | 0,40 |
| Aqua | ad 100 | ad 100 | ad 100 |

21. Beispiele für Zusammensetzungen zum Tränken von Tüchern

| | Bestandteile | 1 | 2 | 3 | 4 |
|---|---|---|---|---|---|
| Phase 1 | PEG-40 Hydrogenated Castor Oil | 0,1 | 0,1 | 0,1 | - |
| | Trideceth-9 | 0,1 | 0,1 | 0,1 | - |
| | Parfum | 0,3 | 0,1 | 0,1 | 0,1 |
| Phase 2 | Hexyllaurat | 2,0 | - | - | - |
| | Dicaprylylcarbonat | - | - | 2,0 | - |
| | Dipropylenglycol | - | 2,0 | - | - |
| Phase 3 | Allantoin | 0,5 | - | - | - |
| | Citronensäure (Monohydrat) | 1,5 | 1,5 | 1,5 | 1,5 |
| | Trinatriumcitrat (Dihydrat) | 2,0 | 2,0 | 2,0 | 2,0 |
| | Decylglucosid | - | - | - | 2,0 |
| | Pemulen TR 1 | - | - | - | 0,2 |
| | Dmt-(D-Arg)-Phe-Lys-NH₂ | 0,03 | 0,02 | 0,003 | 0,01 |
| | Ridulisse C | 1,00 | 0,50 | 1,00 | - |
| | Carnitin | 0,20 | - | - | 0,50 |
| | Wasser | ad 100 | ad 100 | ad 100 | ad 100 |

Mit diesen Tränkzusammensetzungen wurden verschiedene Viskose-Vliesstoffe ausgerüstet. Es wurden gelochte, ungelochte, genoppte, einlagige, zweilagige und dreilagige Vliese ausgerüstet. Die Tücher wurden sowohl als feuchte Tücher als auch als trockene Tücher (das heißt, nach dem Ausrüsten wurden die Tücher bis auf einen Restwassergehalt kleiner 10 Gew.-%, bevorzugt kleiner 5 Gew.-%, getrocknet, um direkt vor Gebrauch mit einer kleinen Menge an Wasser wieder befeuchtet zu werden oder auf feuchter Haut angewendet zu werden) verwendet.

**Matrixpflaster mit schützendem Effekt**

| | 1 | 2 | 3 | 4 | 5 |
|---|---|---|---|---|---|
| DURO-TAK^{®} 387-2516 | 76 | 76 | 76 | 76 | 76 |
| Aloe Vera Gel | 1 | - | 1 | - | - |
| Propylenglycolmonooleat | 5 | 5 | - | - | - |
| Tween^{®}-80 | - | - | 5 | 3 | 5 |
| Natriumcitrat | 0,1 | 0,1 | 0,1 | 0,1 | 0,1 |
| Controx^{®} KS | 0,05 | 0,05 | 0,05 | 0,05 | 0,05 |
| (D-Arg)-Dmt-Lys-Phe-NH₂ | 0,03 | 0,03 | 0,03 | 0,01 | 0,03 |
| Ridulisse C | 1,00 | 0,50 | 1,00 | - | - |
| Carnitin | 0,20 | - | - | 0,50 | 0,30 |
| Matrixyl 3000 | 3,00 | - | - | - | 2,00 |
| Wasser | ad 100 | ad 100 | ad 100 | ad 100 | ad 100 |

**Reservoirpflaster mit schützendem Effekt**

| Bestandteile des Wirkstoffreservoirs | 1 | 2 |
|---|---|---|
| (D-Arg)-Dmt-Lys-Phe-NH₂ | 0,02 | 0,03 |
| Ultrasomes | 1,0 | 1,0 |
| Ridulisse C | - | 0,50 |
| Carnitin | 0,50 | - |
| Ederline H | 1,0 | 1,0 |
| Carbopol^{®} 980 | 0,5 | - |
| Pemulen^{®}TR 1 | - | 0,5 |
| NaOH | 0,1 | 0,1 |
| Titandioxid | 0,2 | 0,2 |
| Ethanol | 1,0 | 1,0 |
| Polyvinylalkohol | 2,0 | 1,0 |
| Carboxymethylcellu lose | 1,0 | 0,5 |
| Glycerin | 10 | 20 |
| Sorbitol | 7,0 | 5,0 |
| 1,3-Butandiol | 3,0 | 3,0 |
| Tween^{®}-80 | 0,05 | 0,05 |
| Paraffinöl | 5,0 | 2,0 |
| Natriumcitrat | 0,1 | 0,1 |
| Controx KS | 0,05 | 0,05 |
| Wasser | ad 100 | ad 100 |

Die Bestandteile der Wirkstoffreservoir-Rezepturen Nr. 1 bzw. 2 wurden miteinander vermischt, so dass eine gelartige Masse entstand. Mit dieser Masse wurden Pflasterträger beschichtet, so dass sogenannte Reservoirpflaster erhalten wurden. Diese Pflaster werden sowohl auf die trockene Haut als auch auf die angefeuchtete Haut gelegt und verbleiben dort für eine Zeit von wenigen Sekunden bis einigen Stunden.

Die nachfolgendenRezepturen sind bevorzugte Beispiele für erfindungsgemäße schützende Körpercremes:

| | | |
|---|---|---|
| Paraffinum Liquidum | 20,00 | 20,00 |
| Arlacel 165 | 4,00 | 4,00 |
| Eutanol G | 1,00 | 1,00 |
| Cetiol SN | 1,50 | 1,50 |
| Cremophor A 6 | 2,00 | 2,00 |
| Behenylalkohol | 1,60 | 1,60 |
| Silikonöl 350 cs | 3,00 | 3,00 |
| Brij 721 VP | 0,40 | 0,40 |
| Propylparaben | 0,20 | 0,20 |
| Controx KS | 0,25 | 0,25 |
| Propandiol-1,2 | 3,00 | 3,00 |
| Glycerin 86% pflanzlich | 5,00 | 5,00 |
| Polyethylenglykol MG 400 | 1,70 | 1,70 |
| Methylparaben | 0,20 | 0,20 |
| Phenoxyethanol, rein | 0,50 | 0,50 |
| Carbopol ETD 2020 | 0,50 | 0,50 |
| Natriumhydroxid | 0,03 | 0,03 |
| Citronensäure Monohydrat | 0,10 | 0,10 |
| Trinatriumcitrat Dihydrat | 0,10 | 0,10 |
| (D-Arg)-Dmt-Lys-Phe-NH₂ | 0,03 | 0,03 |
| Sensiva SC 50 (Octoxyglycerin) | 0,50 | 0,50 |
| Carnitin | 0,20 | 0,20 |
| Ergothionein | - | 0,10 |
| Parfum | 0,30 | 0,30 |
| Simulgel NS | 1,00 | 1,00 |
| Wasser | ad 100,00 | ad 100,00 |

**Liste der verwendeten Rohstoffe**

| Handelsname | INCl-Bezeichnung | Lieferant/Hersteller |
|---|---|---|
| | | |
| Abil EM 90 | CETYL PEG/PPG-10/1 DIMETHICONE | Degussa |
| Argireline | Acetyl-Hexapeptide-3 | Lipotec |
| Arlacel 165 | Glyceryl Stearate, PEG-100 Stearate | Uniqema |
| Aristoflex AVC | Ammonium Acryloly-Dimethyltaurat/VP Copolymer, t-Butyl-Alcohol | Clariant |
| Baysilone-Öl M 350 | Dimethicone | GE Bayer Silicones |
| Belsil DM 100 | Dimethicone | Wacker |
| Brij 721 | STEARETH-21 | Uniqema |
| Calmosensine | BUTYLENE GLYCOL, WATER, LAURETH-3, HYDROXYETHYLCELLULOSE, Acetyl Dipeptide-1 Cetylester | Sederma |
| Caomint | PROPYLENE GLYCOL, AQUA (WATER), MENTHA PIPERITA (PEPPERMINT) LEAF EXTRACT, THEOBROMA CACAO (COCOA) EXTRACT | Solatia |
| Carbopol 980 | Carbomer | Noveon |
| Carbopol ETD 2020 | Acrylates/C10-30 Alkyl Acrylate Crosspolymer | Noveon |
| Cetiol^{®} 868 | Ethylhexyl stearate (vegetable base) | Cog nis |
| Cetiol^{®} B | Dibutyl Adipate | Cog nis |
| Cetiol^{®} HE | PEG-7 Glyceryl Cocoate | Cognis |
| Cetiol^{®} SB 45 | Butyrospermum parkii (Shea butter) | Cognis |
| Cetiol^{®} SN | Cetearyl Isononanoate | Cognis |
| Controx KS | Tocopherol, Hydrogenated Palm Glycerides Citrate | Cog nis |
| Cremophor A 6 | CETEARETH-6, STEARYL ALCOHOL | BASF |
| Culminal MHPC 100 | Hydroxypropylmethylcellulose | Hercules |
| Cutina CBS | Glyceryl Stearate / Cetearyl Alcohol / Cetyl alpitate / Cocoglycerides im Gewichtsverhältnis 7:1:1:1 | Cog nis |
| Cutina GMS-V | Glyceryl Stearate (Mischung aus Glycerylmono- und distearat) | Cog nis |
| Cutina MD | Glyceryl Stearate (Mischung aus Glycerylmono- und distearat) | Cog nis |
| Dehyton K | AQUA (WATER), COCAMIDOPROPYL BETAINE (29 - 32 Gew.-%) | Cog nis |
| Deliner | Zea Mays (Corn) Kernel Extract, Butylene Glycol, Xanthan Gum | Coletica |
| Dow Corning 1403 Fluid | Cyclomethicone (86 - 88 Gew.-%), Dimethiconol (12 - 14 Gew.-%) | Dow Corning |
| Dow Corning 1501 Fluid | Cyclomethicone (85 - 86 Gew.-%), Dimethiconol (14-15 Gew.-%) | Dow Corning |
| Dow Corning 200 Fluid | Dimethicone | Dow Corning |
| Dow Corning 9040 | Cyclomethicone/ Dimethicone Crosspolymer (87-88%/12-13%) | Dow Corning |
| Dow Corning^{®}245 Fluid | Cyclomethicone | Dow Corning |
| Dry Flo Plus | Aluminium Starch Octenylsuccinate | National Starch |
| DSH CN | Water, Dimethylsilanol Hyaluronate | Exsymol |
| DURO-TAK^{®} 387-2516 | Polyacrylate, Ethylcellulose, Polyvinylpyrrolidon | National Starch and Chemical |
| Ederline H | PEG-40 Hydrogenated Castor Oil, PPG-2-Ceteareth-9, Pyrus Malus (Apple) Fruit Extract | Sporga |
| Ederline L | Hexyldecanol, Pyrus Malus (Apple) Fruit Extract | Seporga |
| Elfacos ST 37 | PEG-45/Dodecyl Glycol Copolymer | Akzo Nobel |
| Eumulgin B 3 | Ceteareth-30 | Cog nis |
| Eutanol G | OCTYLDODECANOL | Cog nis |
| Fucogel 1000 | Biosaccharide Gum-1 | Solatia |
| Glistin | Water, L-Glutamylaminoethyl indole | Exsymol |
| Glucamate Sse-20 | PEG-20 methylglucose sesquistearate | Noveon |
| Herbasol Destillat Salbei | Propylene Glycol, Aqua (Water), Saliva Officinalis (SAGE) Leaf Extract | Cosmetochem |
| Herbasol Destillat Hamamelis | Aqua (Water), Alcohol denat., Hamamelis Virginia, Benzoic Acid, Polysorbate 20, Diethylphthalate, | Cosmetochem |
| Herbasol Destillat weißer Tee | Propylene Glycol, Aqua (Water), Camellia Sinensis Leaf Extract | Cosmetochem |
| Hibiscin^{®} HP-LS-9198 | Water, Hibiscus esculentus Seed Extract, Phenoxyethanol | Laboratoires Serobiologiques |
| Hydagen CMF | Chitosan Glycolate | Cog nis |
| Hydrovance | Wasser, Bis-N,N'-(2-Hydroxyethyl)harnstoff, Harnstoff, Ammoniumlactat | National Starch |
| Keltrol SF | Xanthan Gum | Kelco |
| Keratec Pep | Water, Hydrolyzed Keratin | Croda |
| Kombuchka | Saccharomyces/Xylinum/Black Tea Ferment, Glycerin, Hydroxyethylcellulose | Sederma |
| Lameform TGI | Polyglyceryl-3 Diisostearate | Cog nis |
| Lamesoft^{®} PO 65 | Coco-Glucoside, Glyceryl Oleate, Water | Cog nis |
| LANETTE^{®} 22 | Behenyl Alcohol | Cog nis |
| Liftiline | Aqua, Hydrolyzed Wheat Protein (7 - 10 Gew.-% Aktivsubstanz) | Silab |
| Lipochroman 6 | DIMETHYLMETHOXY CHROMANOL | Lipotec |
| Lipoid S 75-3 | Aqua, Lecithine | Lipoid GmbH |
| Matrixyl | Aqua, Palmitoyl Pentapeptide-3 | Sederma |
| Matrixyl 3000 | Glycerin, Aqua, Butylene Glycol, Carbomer, Polysorbate 20, Palmitoyl Oligopeptide, Palmitoyl Tetrapeptide-1 | Sederma |
| Montanov 68 | Cetearyl Alkohol, Cetearyl Glucoside | Seppic |
| Montanov 202 | Arachidyl Alkohol, Behenyl Alcohol, Arachidyl Glucoside | Seppic |
| Myritol 318 | Caprylic/Capric Triglyceride | Cog nis |
| MYRITOL^{®} PC | PROPYLENE GLYCOL DICAPRYLATE/DICAPRATE | Cog nis |
| Natipide 2 PG | Aqua, Propylene Glycol, Lecithin | Phospholipid GmbH |
| Natrulon RC 50 DG | WATER, CARNITINE, POLYGLYCERIN-10 | Lonza |
| Neo Heliopan AP | Disodium Phenyl Dibenzimidazole Tetrasulfonate | Symrise |
| Novata AB | Cocoglycerides | Cog nis |
| Parsol 1789 | BUTYL METHOXYDIBENZOYLMETHANE | DSM |
| Parsol 340 | Octocrylene | DSM |
| Parsol HS | PHENYLBENZIMIDAZOLE SULFONIC ACID | DSM |
| Parsol SLX | Polysilicone-15 | DSM |
| Pearl Protein Extract | Aqua, Hydrolyzed Conchiolin Protein | Maruzen |
| Pemulen TR 1 | Acrylates/C10-30 Alkyl Acrylate Crosspolymer | Noveon |
| Photosomes | Aqua, Lecithine, Plancton Extract | AGI Dermatics |
| Phytokine | Aqua, Butylene Glycol, HYDROLYZED SOY PROTEIN, Methylparaben, Ethylparaben, Propylparaben (0,4 - 0,8 Gew.-% Aktivsubstanz) | Coletica |
| Plantaren^{®} 1200 | Lauryl Glucoside, ca. 50 % Aktivsubstanz | Cog nis |
| Rhamnosoft | Biosaccharide Gum-2 | Solabia |
| Ridulisse C | Water, HYDROLYZED SOY PROTEIN (3 - 5 Gew.-% Aktivsubstanz) | Silab |
| Ronasphere^{®} LDP | SILICA, Cl 77891 (TITANIUM DIOXIDE), Cl 77491 (IRON OXIDES) | Merck KGaA |
| Sensiva^{®} SC 50 | 2-Ethylhexylglycerinether | Schülke & Mayr |
| Sepigel^{®}305 | Aqua (Water) Polyacrylamide, C₁₃-C₁₄ Isoparaffin, Laureth-7 (Aktivsubstanz Verdickerpolymer ca. 45 - 49 Gew.-%) | SEPPIC |
| Sepilift DPHP | Dipalmitoyl Hydroxyproline | Seppic |
| Sepivinol R | Wine Extract (Polyphenolreicher Extrakt aus Rotwein) | Seppic |
| Silymarin Phytosome | Silybum Marianum Extract and Phospholipids | Indena SpA |
| Simulgel EPG | Aqua (Water)/Sodium Acrylate / Sodium Acryloyldimethyl Taurate Copolymer / Polyisobutene / Caprylyl/Capryl Glucoside | Seppic |
| Simulgel NS | Aqua (Water)/Hydroxyethyl Acrylate / Sodium Acryloyldimethyl Taurate Copolymer / Squalane / Polysorbate 60 | Seppic |
| Sisterna SP 30 C | Sucrosedistearate | Sisterna B.V. |
| Sisterna SP 70 C | Sucrose stearate/palmitate | Sisterna B.V. |
| Softisan 649 | Bis-Diglyceryl Polyacyladipate-2 | Sasol |
| Spirulina 3002 | Water, Algae extract (Spirulina platensis) | Interorgana |
| Stenol 16/18 | Cetearylalkohol | Cog nis |
| Symdiol 68 | 1,2-Octandiol, 1,2-Hexandiol | Symrise |
| Sympatens-BS/080 | Stearic acid ethoxylated (8 EO) | Dr. W. Kolb |
| Synperonic PE L 64 | Poloxamer-184 | Uniqema |
| Tego Carbomer 140 | Carbomer | Degussa |
| Texapon^{®} SB 3 | Aqua, Disodium Laureth Sulfosuccinate, (ca. 40 % Aktivsubstanz), Citric Acid | Cog nis |
| Tween^{®}-80 | Polysorbate-80 | |
| Ultrasomes | Aqua, Lecithine, Micrococcus Luteus Extract | AGI Dermatics |
| Uvinul MBC 95 | 4-METHYLBENZYLIDENE CAMPHOR | BASF |

## Patentansprüche

1. Zusammensetzung, enthaltend
mindestens ein Peptid, ausgewählt aus Peptiden, welche
a) eine Länge von drei bis sechzehn Aminosäuren besitzen und
b) mindestens eine positive Nettoladung (bei pH 7,2) aufweisen und
c) mindestens zwei Aminosäuren mit aromatischen Seitenketten aufweisen, von denen mindestens eine Aminosäure ausgewählt ist aus Tyr, 2'-Methyltyrosin, 6'-Methyltyrosin, 2',6'-Dimethyltyrosin (Dmt), N,2',6'-Trimethyltyrosin (Tmt) oder 2'-Hydroxy-6'-Methyltyrosin (Hmt)
und 0,001 bis 95 Gew.-% Wasser.

2. Zusammensetzung nach Anspruch 1, **dadurch gekennzeichnet, dass** das Peptid eine Länge von vier bis zwölf, bevorzugt von vier bis acht Aminosäuren besitzt.

3. Zusammensetzung nach Anspruch 1 oder 2, **dadurch gekennzeichnet, dass** das Peptid mindestens eine D-Aminosäure enthält.

4. Zusammensetzung nach einem der Ansprüche 1 bis 3, **dadurch gekennzeichnet, dass** die C-terminale Carboxylgruppe der Aminosäure am C-Terminus amidiert ist.

5. Zusammensetzung nach einem der Ansprüche 1 bis 4, **dadurch gekennzeichnet, dass** das Verhältnis der Anzahl der Aminosäuren mit aromatischen Seitenketten zur Anzahl der positiven Nettoladungen des Peptids 0,3 bis 3, bevorzugt 0,6 bis 2 beträgt.

6. Zusammensetzung nach einem der Ansprüche 1 bis 5, **dadurch gekennzeichnet, dass** das Peptid die Aminosäurenfolge Xxx-Lys umfasst, wobei die Aminosäure Xxx ausgewählt ist aus Phe, 2',6'-Dimethylphenylalanin (Dmp), Trp, Tyr, 2'-Methyltyrosin, 6'-Methyltyrosin, 2',6'-Dimethyltyrosin (Dmt), N,2',6'-Trimethyltyrosin (Tmt) oder 2'-Hydroxy-6'-Methyltyrosin (Hmt).

7. Zusammensetzung nach einem der Ansprüche 1 bis 6, **dadurch gekennzeichnet, dass** das Peptid eine alternierende Abfolge von Aminosäuren mit bei pH 7,2 positiv geladenen Seitenketten und Aminosäuren mit aromatischen Seitenketten aufweist.

8. Zusammensetzung nach einem der Ansprüche 1 bis 7, **dadurch gekennzeichnet, dass** das Peptid ausgewählt ist aus Peptiden mit den Aminosäuresequenzen (D-Arg)-Dmt-Lys-Phe-NH₂, Dmt-(D-Arg)-Phe-Lys-NH₂, Tyr-(D-Arg)-Phe-Lys-NH₂, Phe-(D-Arg)-Dmt-Lys-NH₂ und 2',6'-Dmp-(D-Arg)-Dmt-Lys-NH₂, insbesondere (D-Arg)-Dmt-Lys-Phe-NH₂ und Dmt-(D-Arg)-Phe-Lys-NH₂.

9. Zusammensetzung nach einem der Ansprüche 1 bis 8, **dadurch gekennzeichnet, dass** das mindest eine Peptid in einer Konzentration von 0,00001 bis 10 Gew.-%, bevorzugt zu 0,0001 bis 1 Gew.-%, bezogen auf das Gesamtgewicht der Zusammensetzung, enthalten ist.

10. Zusammensetzung gemäß einem der Ansprüche 1 bis 9, **dadurch gekennzeichnet, dass** das mindest eine Peptid in Liposomen verkapselt ist.

11. Zusammensetzung nach einem der Ansprüche 1 bis 10, **dadurch gekennzeichnet, dass** mindestens eine weitere Aktivsubstanz enthalten ist, die ausgewählt ist aus Aporphin-Alkaloiden, Purin und/oder Purinderivaten, natürlichen Betainverbindungen, alkyl- oder hydroxyalkylsubstituierten Harnstoffverbindungen, Monomeren, Oligomeren und Polymeren von Aminosäuren, N-C₂-C₂₄-Acylaminosäuren und/oder den Estern und/oder den physiologisch verträglichen Salzen dieser Substanzen, Polysacchariden, sowie Mischungen dieser Wirkstoffe.

12. Zusammensetzung gemäß einem der Ansprüche 1 bis 11, **dadurch gekennzeichnet, dass** sie zusätzlich mindestens einen weiteren Wirkstoff, ausgewählt aus Retinoiden, insbesondere Retinol und C₂-C₂₂-Fettsäureestern des Retinols, Oleanolsäure, Oleanol, Kreatin, sowie Mischungen der vorgenannten Substanzen enthält.

13. Nicht-therapeutische, kosmetische Verwendung mindestens eines Peptids zur Bekämpfung der Hautalterung, zum Schutz der Haut vor Schäden durch ultraviolette und/oder infrarote Strahlung, zur Verbesserung der Elastizität der Haut und/oder zur Verringerung und/oder Verhinderung der Faltenbildung, **dadurch gekennzeichnet, dass** das Peptid ausgewählt ist aus Peptiden, welche
a) eine Länge von drei bis sechzehn Aminosäuren besitzen und
b) mindestens eine positive Nettoladung (bei pH 7,2) aufweisen und
c) mindestens zwei Aminosäuren mit aromatischen Seitenketten aufweisen, von denen mindestens eine Aminosäure ausgewählt ist aus Trp, Tyr, 2'-Methyltyrosin, 6'-Methyltyrosin, 2',6'-Dimethyltyrosin (Dmt), N,2',6'-Trimethyltyrosin (Tmt) oder 2'-Hydroxy-6'-Methyltyrosin (Hmt).

14. Verwendung mindestens eines Peptids zur Herstellung einer Zusammensetzung zur Bekämpfung der Hautalterung, zum Schutz der Haut vor Schäden durch ultraviolette und/oder infrarote Strahlung, zur Verbesserung der Elastizität der Haut und/oder zur Verringerung und/oder Verhinderung der Faltenbildung, **dadurch gekennzeichnet, dass** das Peptid ausgewählt ist aus Peptiden, welche
a) eine Länge von drei bis sechzehn Aminosäuren besitzen und
b) mindestens eine positive Nettoladung (bei pH 7,2) aufweisen und
c) mindestens zwei Aminosäuren mit aromatischen Seitenketten aufweisen, von denen mindestens eine Aminosäure ausgewählt ist aus Tyr, 2'-Methyltyrosin, 6'-Methyltyrosin, 2',6'-Dimethyltyrosin (Dmt), N,2',6'-Trimethyltyrosin (Tmt) oder 2'-Hydroxy-6'-Methyltyrosin (Hmt).
